# EUROPEAN PATENT SPECIFICATION

(11) **EP 2 680 800 B1**
(45) Date of publication and mention of the grant of the patent: **27.04.2016**
(21) Application number: 12752239.9
(22) Date of filing: 02.03.2012
(51) Int. Cl.: A61F 13/00, A61F 13/02

(54) **DEVICES FOR SKIN TIGHTENING**
VORRICHTUNGEN ZUR HAUTSTRAFFUNG
DISPOSITIFS PERMETTANT LE RESSERREMENT DE LA PEAU

(30) Priority: 03.03.2011 US 201161448809 P; 15.04.2011 US 201161476163 P; 01.03.2012 US 201261605717 P
(43) Date of publication of application: 08.01.2014
(73) Proprietor: Neodyne Biosciences, Inc., Menlo Park, CA 94025 (US)
(72) Inventor: GURTNER, Geoffrey, C., Palo Alto, CA 94306 (US); LONGAKER, Michael, T., Atherton, CA 94027 (US); DAUSKARDT, Reinhold, H., Menlo Park, CA 94025-5647 (US); HORNE, Kenneth, N., San Francisco, CA 94123 (US); MEHTA, Bankim, H., San Ramon, CA 94582 (US); ROSENTHAL, Michael, H., Menlo Park, CA 94025 (US); RIMSA, Joseph, Palo Alto, CA 94303 (US); SALINAS, Sergio, Redwood City, CA 94063 (US); HARRIS, Melanie, Santa Clara, CA 95054 (US); SPOONER, Greg, Menlo Park, CA 94025 (US); CHAN, Kin, Menlo Park, CA 94025 (US); JACKSON, Jasper, Newark, CA 94560 (US); ZEPEDA, John, A., Los Altos, CA 94024 (US); BEASLEY, William, R., Los Altos, CA 94022 (US); YOCK, Paul, Atherton, CA 94027 (US); ICHIRYU, Keiichiro, Campbell, CA 95008 (US); CARDONA PAMPLONA, Manuel, A., Calabasas, CA 94303 (US); KROG, Tor, C., Seattle, WA 98115 (US); LEVI, Kemal, Mountain View, CA 94043 (US); CAO, Michael, T., Milpitas, CA 95035 (US); RODRIGUEZ, Rene, Santa Clara, CA 95054 (US)
(74) Representative: Roberts, Mark Peter
(86) International application number: PCT/US2012/027618
(87) International publication number: WO 2012/119131

(56) References cited:
- EP-A1- 2 161 011
- WO-A2-2011/019859
- US-A- 633 050
- US-A- 3 613 679
- US-A- 4 653 492
- US-A- 4 753 232
- US-A1- 2006 282 135
- US-A1- 2007 142 761
- US-A1- 2008 208 098
- US-B1- 6 264 976

## Description

The present invention relates generally to a skin treatment device, which may be used for improved healing of skin after a therapeutic injury. For example, such a device can be used to produce improved tightening of skin after a therapeutic treatment. The device can also be used to produce a temporary cosmetic effect by displacing skin to stimulate a clinical effect.

Many procedures involve producing a therapeutic injury to tissue to produce an improved therapeutic or cosmetic effect in the tissue. Skin tightening is one such therapy that involves creating an injury to produce an improved cosmetic appearance of the skin near or around the site of the therapeutic injury. Skin tightening can be performed many different ways ranging from invasive treatments to less invasive procedures such as IPL (intense pulsed light). Typically invasive procedures appear to be the most effective but require significant recuperative periods, while less invasive procedures are less effective than invasive procedures but the recuperative periods are shorter. In any case, the heating process that occurs subsequent to the therapeutic injury can determine the effectiveness of the procedure.

Again, in referring to skin tightening as one example, during the procedure a physician or medical practitioner induces a controlled trauma in the skin. This is typically performed by applying energy to the tissue to either ablate (vaporize) or non-ablatively heat the skin to create either patterns of lesions or a localized area of treatment. There are limits when creating therapeutic injury to tissue, if there is variability in the lesion or hole created in the tissue the healing process may not produce the optimal effect. For example, if the therapeutic treatment creates openings in the tissue that are too large, the tissue may not heal as desired.

US 6,264,976 B1 discloses an absorbent pad dressing frame delivery system.

A need remains for devices to improve the outcome of such medical procedures by improving the healing process of the tissue subsequent to the treatment.

According to the present invention, a skin treatment device is provided, as defined in the independent claim. The first layer may comprise at least one elastic material opening through the elastic material configured to be aligned with the at least one opening of the second layer for treatment through the aligned openings. The brace may further comprise a mating element configured to secure the first layer in a strained configuration on the brace. The brace may be rigid. The brace may comprise a plurality of segments bendable with respect to another of the plurality of segments to provide a variable contour of the brace. The brace may be configured to be bendable in a plurality of directions. Each of the plurality of segments may be configured to be bent in a plurality of directions. The second layer may comprise a mask having a pattern of openings. The pattern of openings comprises a treatment pattern. The skin treatment device may further comprise a connection element configured to connect the device to an energy emitting skin treatment device. Each second layer opening may correspond to at least one individual treatment zone. The second layer may be removable from the first layer after treatment is provided through the at least one second layer opening.

A skin treatment system is disclosed, comprising an dressing with a load per millimeter width of at least 0.1 Newtons at a strain of at least 0.01,0.05,0.1,0.2,0.3,0.4,0.5,0.6,0.7,0.8,0.9 or higher, and a limiting member coupled to the dressing and configured to resist straining of the dressing beyond a predetermined strain. The limiting member may comprise a first handle at a first end of the at least one limiting member, and a second handle at a second end of the at least one limiting member. The skin treatment system may comprise at least two elongate strain limiting structures. The first handle may be contiguously or non-contiguously coupled to the dressing between the first ends of the at least two elongate strain limiting structures. The second handle may also be contiguously or non-contiguously coupled to the dressing between the second ends of the at least two elongate strain limiting structures. The predetermined strain may be at least 0.01,0.05,0.1,0.2,0.3,0.4,0.5, 0.6, 0.7, 0.8, 0.9 or higher. The limiting member may be releasably coupled to the dressing. The limiting member may be adhered to the dressing using an adhesive. The adhesive may comprise a shear-resistance to a force level that is greater than the T-peel resistance to the force level. The first handle and the second handle may comprise a substantially inelastic material relative to the dressing, which may optionally be a semi-rigid or rigid material. The limiting member may comprise at least one flexible, inelastic elongate element. The dressing may comprise an unstrained configuration in which a distance between a first attachment region of the limiting member and a second attachment region of the limiting member is less than a length of the limiting member between the first attachment region and the second attachment region, and may comprise a strained configuration at the predetermined strain wherein the distance between the first attachment region of the limiting member and a second attachment region of the limiting member is substantially equal to the a length of the limiting member between the first attachment region and the second attachment region. The limiting member may comprise a folded board with at least three two folds, or a ratchet and pawl mechanism. The limiting member may be selectively configured to resist straining of the dressing beyond a plurality of predetermined strains. The plurality of predetermined strains may comprise graphical indicia on the limiting member.

A skin treatment system is disclosed, comprising an dressing, comprising a tensioning axis, and a limiting member coupled to the dressing and configured to resist straining of the dressing beyond a predetermined strain, wherein the attachment of a first end of the limiting member to the dressing is contiguous across a dimension of the dressing transverse to the tensioning axis. The dressing may have a load per millimeter width of at least 0.1 Newtons at a strain of at least 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or higher. The limiting member may comprise a first handle at a first end of the at least one limiting member, and a second handle at a second end of the at least one limiting member. The skin treatment system may comprise at least two elongate strain limiting structures. The first handle may be contiguously coupled to the dressing between the first ends of the at least two elongate strain limiting structures. The second handle may also be contiguously coupled to the dressing between the second ends of the at least two elongate strain limiting structures. The predetermined strain may be at least 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or higher. The limiting member may be releasably coupled to the dressing. The limiting member may be adhered to the dressing using an adhesive. The adhesive may comprise a shear-resistance to a force level that is greater than the T-peel resistance to the force level. The first handle and the second handle may comprise a substantially inelastic material relative to the dressing, which may optionally be a semi-rigid or rigid material. The limiting member may comprise at least one flexible, inelastic elongate element. The dressing may comprise an unstrained configuration in which a distance between a first attachment region of the limiting member and a second attachment region of the limiting member is less than a length of the limiting member between the first attachment region and the second attachment region, and may comprise a strained configuration at the predetermined strain wherein the distance between the first attachment region of the limiting member and a second attachment region of the limiting member is substantially equal to the a length of the limiting member between the first attachment region and the second attachment region. The limiting member may comprise a folded board with at least three two folds, or a ratchet and pawl mechanism. The limiting member may be selectively configured to resist straining of the dressing beyond a plurality of predetermined strains. The plurality of predetermined strains may comprise graphical indicia on the limiting member.

A skin treatment system is disclosed, comprising an elastic structure, first and second handles attached to opposite regions of the elastic structure, wherein the first and second handles are coupled to the elastic structure and configured to provide a substantially uniform tensile force across the elastic structure; and a strain indicator. The strain indicator may comprises graphical or numerical indicia of the degree of strain.

A multilayered elastic dressing is disclosed, comprising a plurality of elastic layers, wherein each layer removably coupled to another layer of said plurality of elastic layers; wherein said plurality of elastic layers comprises a base layer having a skin adhesive layer on a skin adhesive side of the base layer and at least one additional layer. Each said at least one additional elastic layer may be removable from the base layer after the dressing has been strained and adhered to skin of a subject to thereby selectively alter the stresses placed on the skin through the base layer.

A method of treating a subject is disclosed, comprising creating a plurality of lesions on a subject's skin and placing a dressing over the lesions on the subject's skin.

A method treating a subject is disclosed, comprising placing a dressing over a skin region and creating lesions in the skin region through the dressing. Creating lesions in the skin regions may be performed using an energy-based modality. The dressing may comprise a mask region configured to selectively block the energy-based modality and a treatment region configured to selectively permit energy from the energy-based modality to pass through the dressing. The treatment region comprises an uninterrupted structure configured to selectively permit energy to pass through the uninterrupted structure. The uninterrupted structure may be an optically clear structure. The method may further comprise aligning a treatment device to indicia located on the dressing. The method may further comprise aligning a treatment device to pre-existing openings in the dressing. The pre-existing openings may be pre-existing potential openings in the dressing. The pre-existing potential openings may comprise slits. The method may further comprise creating a plurality of openings in the dressing using a treatment device.

A method of treating a subject is disclosed, comprising maintaining a strain in an elastic dressing, placing the strained dressing over skin of a subject, creating at least one lesion on the skin of the subject through the dressing; and then releasing the dressing so that compressive forces from the dressing are applied to the skin of the subject.

The invention will now be described, by way of example, with reference to the accompanying drawings.
Figs. 1A to 1C illustrate the process of a conventional skin tightening procedure performed on a section of skin.
Fig. 2A illustrates an example of a wound dressing that assists in the healing process to improve the outcome of a cosmetic procedure or provides temporary movement of tissue.
Figs. 2B to 2D show an example of a dressing assisting in a wound healing process.
Figs. 2E to 2G show another example of application of a dressing to assist in the wound healing process.
Figs. 2H and 2I illustrate an example of a second dressing or frame placed upon an initial dressing.
Figs 3A and 3B respectively show an expanded or strained dressing and a dressing in an unstrained or relaxed profile.
Figs. 3C to 3F show additional variations of dressings.
Figs. 3G and 3H respectively depicted a dressing with slit openings in an unstrained and strained profile.
Figs. 3I and 3J depict additional variations of dressings with reinforced openings.
Figs. 4A and 4B illustrates a dressing and frame respectively, where the frame maintains the dressing in a strained configuration for eventual placement on tissue.
Fig. 4C shows the dressing of Fig. 4A positioned on the frame of Fig. 4B.
Fig. 4D shows a cross sectional view taken along the line 4D-4D of Fig. 4C.
Fig. 4E illustrates an optional applicator used to assist in positioning the dressing of Fig. 4A from a frame to tissue.
Fig. 4F depicts a dressing with reinforced retention openings.
Figs. 5A to 5C illustrate another variation of a dressing according to the present disclosure.
Figs. 6A and 6B shows a dressing having one or more limiting members to pre-determine a strain capacity of the dressing.
Figs. 6C and 6D illustrate a variation of a dressing that incorporates a bio-active substance that is intended for delivery to or near the site of the lesion.
Fig. 6E and 6F show a variation of a dressing having a varying adhesive pattern on the dressing.
Figs. 6G and 6H show a dressing that has regions of varying elasticity or stretchability.
Figs. 6I to 6K show additional variations of dressings that provide directional or vectored application of force.
Fig. 7A illustrates a contoured shape multi-layer dressing having regions of varying thickness to allow for customization of a strain amount of the dressing.
Fig. 7B illustrates a further variation of a dressing that includes a thermally responsive material.
Figs. 7C and 7D illustrate various mechanisms for application of a dressing.
Figs. 8A and 8B show a dressing positioned adjacent to treatment devices that are used to create lesions within tissue.
Figs. 9A and 9B show an adjustable frame that permits shaping of the frame to adjust to a contour of a surface of tissue.
Fig. 9C shows a variation of a frame employing an expandable member or bladder to compress tissue within the perimeter of the bladder.
Fig. 9D illustrates another example of an adjustable frame that is used to compress tissue or expand a dressing.
Figs. 10A to 10C illustrate a settable adhesive for use with dressings of the present disclosure.
Fig. 11A illustrates an exemplary plot of power versus time for a laser treatment application so that a single laser can create an opening in a dressing and a subsequent lesion at the site of the opening.
Figs. 11B to 11E illustrate another variation of using multiple laser sources to create lesions in solid dressing.
Figs. 12A and 12B shows an example of a dressing having registration features that allow for detection by the treatment device.
Figs. 13A to 13E show another variation of a dressing covered by a mask that directs laser energy to openings within the dressing to control placement of lesions.
Figs. 14A to 14D illustrate various types of lesions that can be created in situ with a solid dressing.
Figs. 15A to 15D illustrate another use for dressings of the present disclosure.
Fig. 16 depicts an additional use of a dressing as a mask to direct creation of a lesion.
Figs. 17A and 17B provides a top view to demonstrate compression or application of force to a lesion.
Figs. 18A to 18F show variations of shaped lesions having geometries to prevent high stress areas when compressed.
Figure 19A is a top view of a skin treatment device in a first configuration.
Figure 19B is a top view of the skin treatment device of Figure 19A in a second configuration.
Figure 20A is a top perspective view of a skin treatment device in a first configuration. Figure 20C is a side elevational view of the skin treatment device in Figure 20A.
Figure 20B is a top perspective view of a skin treatment device in a strained second configuration.
Figure 21A is a top perspective view of a skin treatment device in a relatively unstrained configuration.
Figure 21B is a top perspective view of the skin treatment device of Figure 20A in a first strained configuration.
Figure 21C is a top perspective view of the skin treatment device of Figure 20A in a second strained configuration.
Figure 22A is a top schematic view of a skin treatment device in a first configuration.
Figure 22B is a top schematic view of the skin treatment device of Figure 22A in a second strained configuration.
Figure 23A is a top perspective view of a skin treatment device in a first configuration.
Figure 23B is a top perspective view of the skin treatment device of Fig. 5A in a second configuration.
Figure 24A is a top perspective view of a skin treatment device in a first configuration.
Figure 24B is a top perspective view of the skin treatment device of Figure 24A in a second, strained configuration.
Figure 25A is a top perspective view of a skin treatment device in an unstrained configuration.
Figure 25B is a bottom perspective view of the skin treatment device of Figure 7A in the unstrained configuration.
Figure 25C is a top perspective view of a skin treatment device of Figure 25A in a strained configuration.
Figure 25D is a bottom perspective view of the skin treatment device of Figure 25C in the strained configuration.
Figure 26A is a top perspective view of a skin treatment device in an unstrained configuration.
Figure 26B is a top perspective view of the skin treatment device of Figure 26A in a strained configuration.
Figure 27A is a top perspective view of a skin treatment device in a first configuration.
Figure 27B is a top perspective view of the skin treatment device of Figure 27A in a second strained configuration.
Figure 28A is a top perspective view of a skin treatment device in a first configuration.
Figure 28B is a top perspective view of the skin treatment device of Figure 28A in a second configuration.
Figure 28C is a top perspective view of the skin treatment device of Figure 28A in a third strained configuration.
Figure 28D is a top perspective view of the skin treatment device in a first configuration.
Figure 28E is a top perspective view of the skin treatment device of Figure 29D in a second strained configuration.
Figure 28F is a top perspective view of the skin treatment device of Figure 28D in a third strained configuration.
Figure 28G is a top perspective view of the skin treatment device of Figure 28D in a fourth strained configuration.
Figure 29A is top schematic view of a skin treatment device in a first configuration.
Figure 29B is a bottom schematic view of the skin treatment device of Figure 29A in the second configuration.
Figure 29C is a top schematic view of the skin treatment device of Figure 29A in a second, strained configuration.
Figure 29D is a bottom schematic view of the skin treatment device of Figure 29A in a second, strained configuration.
Figure 30A is top schematic view of a skin treatment device in a first configuration.
Figure 30B is a bottom schematic view of the skin treatment device of Figure 30A in the second configuration.
Figure 30C is a top schematic view of the skin treatment device of Figure 30A in a second, strained configuration.
Figure 30D is a bottom schematic view of the skin treatment device of Figure 30A in a second, strained configuration.
Figure 31A is a top perspective view of a skin treatment device in a first configuration.
Figure 31B is a top perspective view of the skin treatment device of Figure 31A in a second, partially strained configuration.
Figure 31C is a top perspective view of the skin treatment device of Figure 31A in a third, strained configuration.
Figure 32A is a top perspective view of a skin treatment device in a first configuration.
Figure 32B is a top perspective view of the skin treatment device of Figure 32A in a second, strained configuration.
Figure 32C is a top perspective view of the skin treatment device in a first configuration.
Figure 32D is a top perspective view of the skin treatment device of Figure 32C in a second, strained configuration.
Figure 33A is a side perspective view of a tensioning device and skin treatment device prior to straining.
Figure 33B is a side perspective view of the tensioning device of Figure 33A straining skin treatment device of Figure 33B.
Figure 34A is a side elevated view a dressing and tensioning device.
Figure 34B is a top view of the dressing of Figure 34A.

It is believed that controlling, managing or modulating stresses acting in and/or on skin ("mechanomodulation") may have beneficial effects. Modulation of mechanical stresses or effects acting in and/or on skin may translate into or induce biomechanical response, including but not limited to, responses relating to scarring, scar proliferation or other effects.

Devices, methods, systems and kits described herein may relate to devices used to shield skin or a wound from its mechanical environment. The term "shield" is meant to encompass the unloading of stress experienced by the skin or wound as well as and/or providing a physical barrier against contact, contaminants, and the like. The stress shielding or force offloading devices and methods described here may shield the skin or a wound by unloading endogenous stress and/or exogenous stresses. In some variations, the devices may shield the skin from endogenous stress without affecting exogenous stress on the skin, e.g., devices that modify the elastic properties of the skin, etc. In other variations, the devices may shield the wound from exogenous stress without affecting endogenous stress on the skin wound. In still other variations, the devices shield the skin from both endogenous and exogenous stress.

Devices, kits and methods described herein may treat skin at a skin site ("skin treatment device"), including without limitation, to ameliorate the formation of scars at wound sites by controllably stressing or straining the epidermis and deeper layers of dermal tissue at or near a skin site, i.e., at or adjacent a wound or treatment site of a subject's skin, thereby reducing tensile or compressive stress at the skin site. The stress at the skin site may be reduced to levels below that experienced by normal skin and tissue. The stress or strain may be applied to surrounding tissue in one, two, or more directions to reduce endogenous or exogenous stress at the skin site in one, two or more directions. Thus, devices and methods described herein may reduce the stress experienced by skin and/or a wound and surrounding tissues in order to treat a subject. The device may also assist in preventing or reducing the incidence of wound dehiscence.

"Dressing" or "Skin Device" as used herein may include but is not limited to, a skin treatment device, wound treatment device, scar or keloid treatment device, scar or keloid amelioration or prevention device, bandage, or dressing, that may be applied, attached to or coupled to one or more layers of the skin or tissue of a subject.

Devices, kits and methods described herein may be for the preparation and/or application of a dressing. Such preparation may include but is not limited to, for example, removal of an adhesive liner, straining or tensioning a dressing, orienting a dressing for application and/or applying a medicament or other material to a portion of the dressing prior to application.

According to some variations, the packaging, tensioning device, dressing carrier, support, base, handles, manipulation elements and/or applicator may further comprise an opening, a window, or a clear or semi-opaque portion through which a wound, incision or other location may be visualized as the dressing is applied to the skin. According to some variations, the window guides the application of a dressing so that there is an optimal or desired distance between the wound and the edges of the dressing and/or so that the dressing is in an optimal location for unloading skin stresses.

According to some variations, a packaging, manipulation element, and/or applicator is more rigid or provides sufficient column strength in at least a first direction to be supportive of a dressing, while being relatively more flexible and less rigid in at least second direction to provide for a more conforming application to a curved or shaped skin surface of a subject or to permit curvature or shaping of the dressing where it is applied. The first and second directions may or may not be orthogonal to each other. According to some variations, a packaging applicator, tensioning device or dressing carrier, support or base is sufficiently rigid or supportive of a dressing while permitting shaping of the dressing. According to some variations, the carrier or support which may include a base and/or a cover may comprise segments of relatively more rigid material flexibly coupled to adjacent segments to provide flexibility to permit shaping of packaging/applicator and/or dressing while providing sufficient support of the dressing during application. According to some variations, segments are coupled to adjacent segments by way of a flexible material, such as a low-density polyethylene (LDPE) material, or a composite of adhesive and a thinner more flexible substrate. Alternatively, segments may be formed as a structure by manufacturing a substrate with cut-outs, slots, grooves, scoring or other openings or variations in thickness of the substrate at different locations.

The packaging, applicator, manipulation elements tensioning device, or dressing carrier may have elements or features the provide flexibility in one direction while limiting flexibility in another direction. Each of the elements may permit flexing in a different direction than one or more of the other elements. Flexible elements may be straight, or shaped according to a desired application or location of placement. According to some variations, the flexible elements may limit flexibility when the device is being strained and permit flexibility when the device is being applied to the skin.

According to variations, flexible elements are provided in combination with support elements that provide sufficient support to allow a user to maintain the dressing in a strained configuration. According to variations, one or more elements may be provided to maintain a strained dressing in a strained configuration, for example a securing element that secures the dressing in a strained configuration until it is applied to a subject and is released from the carrier, support, base, manipulation element, tensioning device or applicator. For example, after straining the dressing, the dressing may be adhered or attached to one or more elements of a dressing, support, base, manipulation elements, tensioning device or applicator or dressing assembly until it is released from the carrier, support, base tensioning device or applicator or assembly.

The methods, procedures, kits, and devices described herein are intended to assist with the healing process of tissue that was previously or simultaneously treated for a therapeutic or cosmetic effect. Assisting in the healing process can produce an improved outcome and in some cases can eliminate or reduce variability with the healing process. The combined therapeutic process of treating tissue and assisting the ensuing healing process can produce a consistent skin tightening procedure and improve the outcome of the procedure. It is noted that combinations of variations of the methods, kits, and/or procedures as well as combination of specific aspects of methods, kits, and/or procedures are within the scope of this disclosure even though such embodiments may not be specifically shown.

Devices, kits and methods described herein may be for treatment of a subject at a skin site including without limitation for wound treatment or the treatment, amelioration, or prevention of scars and/or keloids, by manipulating mechanical or physical properties of skin or by shielding skin from stresses, and/or by controllably stressing or straining the epidermis and layers of dermal tissue at or near a skin site, i.e., at or adjacent a wound or a treatment site of a subject's skin. According to variations, manipulating mechanical or physical properties may thereby modulate tensile or compressive stress at the skin site. The stress at the skin site may be reduced to levels below that experienced by normal skin and tissue. The stress at the skin site may be increased to levels above that experienced by normal skin and tissue. The stress or strain may be applied to surrounding tissue in one, two, or more directions to manipulate endogenous or exogenous stress at the skin site in one, two or more directions. According to variations, devices and methods described herein may reduce or otherwise manipulate the stress experienced by skin and/or a wound and surrounding tissues in order to treat a subject. The devices may also assist in preventing or reducing the incidence of wound dehiscence.

According to the devices, kits and methods described herein, a skin treatment device, skin device, wound treatment device, scar or keloid treatment device, scar or keloid amelioration or prevention device, bandage, or dressing may be provided that may be applied, attached to or coupled to one or more layers of the skin or tissue of a subject (hereinafter referred to as "dressing", "skin device" or "skin treatment device").

U.S. Patent 7,683,234 to Gurtner et al describes devices and methods intended for the amelioration of scar and/or keloid formation and include a discussion of wound healing as well other information that can be combined with the novel devices of the present invention.

The present disclosure describes the methods, procedures, kits, and devices for use with skin tightening, treatment of skin laxity, skin contraction, skin shrinkage, and maybe even body sculpting procedures for purposes of illustration. The benefits of the present disclosure can be applied in any number of medical procedures requiring providing augmentation of the healing process of a therapeutic or cosmetic treatment. For example, the devices herein may be used for sutureless wound closure, skin splinting or other supportive uses. Other uses for these skin treatment devices may or may not include without limitation, for example, amelioration or prevention of scar formation, treating skin related conditions such as acne, blemishes, rosacea, warts, rashes (including but not limited to erythematous, macular, papular and/or bullous conditions), psoriasis, skin irritation/sensitivity, allodynia, telangiectasia, port wine stains and other arterio-venous malformations, and ectopic dermatitis; treating or improving existing scars, wrinkles, stretch marks, loose or sagging skin or other skin irregularities; lifting, pinning, holding, moving skin for various purposes such as during pre-operative preparation, during surgical procedures for example as a low-profile tissue retractor, to stabilize blood vessels during needle or catheter insertion, postoperatively, pre or post operatively for pre-treating or preconditioning skin for example, prior to scar revision, wound incision, body contouring, in mastectomy skin expansion, during weight loss, or for aesthetic purposes; hair removal or hair loss; treating and/or closing skin injuries for example, incisions, wounds, chronic wounds, bed sores, ulcers (including venous stasis ulcers), preventing or reducing the incidence of wound dehiscence, diabetic skin or wound conditions, burn healing and/or relief; acting as an occlusive or negative-pressure wound dressing; protecting incisions or wounds, e.g. prevention of splitting or opening, protecting newborn belly buttons after cutting umbilical cord. Such treatments may include use of a drug or other therapeutic agent that may be applied to the skin with such device. The agents may include but are not limited to antibiotics, anti-fungals, immune modulators including corticosteroids and non-steroidal immune modulators. The agents may be provided in any of a variety of formulations, including but not limited powders, gels, lotions, creams, pastes, suspensions, etc. The devices may also be used for purposes of delivering a drug to the skin or through the skin, for example by stretching the skin and applying a drug thereto. Different configurations of the device may be amenable to the size or geometry of different body regions. The treatments may be applied to regions of any shape (e.g. linear, curved, stellate), size or depth, and to one or more regions of the body, including but not limited to the scalp, forehead, face (e.g. nose, eyelid, cheeks, lips, chin), ears, neck, shoulder, upper arm, lower arm, palm, dorsum of the hand, fingers, nailbed, axilla, chest, nipple, areola, back, abdomen, inguinal region, buttocks, perineal region, labia, penis, scrotum, thigh, lower leg, plantar surface of the foot, dorsal surface of the foot, and/or toes.

A number of procedures for tightening of skin are commonly known. One non-invasive approach involves the use of laser energy to ablate (vaporize) or non-ablatively heat the skin. Ablative procedures are generally more invasive (i.e. longer down time) and effective. These types of lasers do not produce consistent skin tightening, and presently none of these lasers are FDA indicated for skin tightening. In general, the procedure relies on a lesion (if an area is treated) or a number of lesions to heal after the injury to produce a tightened appearance in or around the skin that was treated. However, if the holes or lesion made by the treatment device are too large, the skin surface will not heal well. In addition, the procedure might not produce an optimal outcome if the healing process is not consistent between the lesions. Furthermore, care must be taken to prevent environmental factors from interfering with the healing process. Additionally many non-ablative tightening procedures rely on collagen contraction to produce volume changes in the collagen by a thermal denaturation process that produces dimensional changes in skin in one or more axes. See, for example, the RF product THERMAGE® (Solta Corp., Hayward, CA) or the IR product TITAN® (Cutera, Inc., Brisbane, CA). The devices herein may also be used with other skin treatments (aesthetic or not) or resurfacing procedures whether topical or subdermal, whether or not using an energy modality such as, for example, microwave, radiofrequency ablation, high-intensity focused ultrasound, laser, infrared, incoherent light, thermal (heat and/or cold, ablative or non-ablative), use of vacuum or suction, vibration or massage (e.g. ENDERMOLOGIE®, LPG Systems, France). The methods, kits, and devices described herein can optionally be used with such non-ablative tightening procedures as well. For example, a water jet can be used to create lesions by directing water or other liquid, and/or a mix of liquid and particles to create the lesions. Furthermore, the water or fluid can be used to swell or expand tissue. Once expanded the tissue can be treated such that upon reversion to a normal non-swelled state, the tissue engages in natural compression.

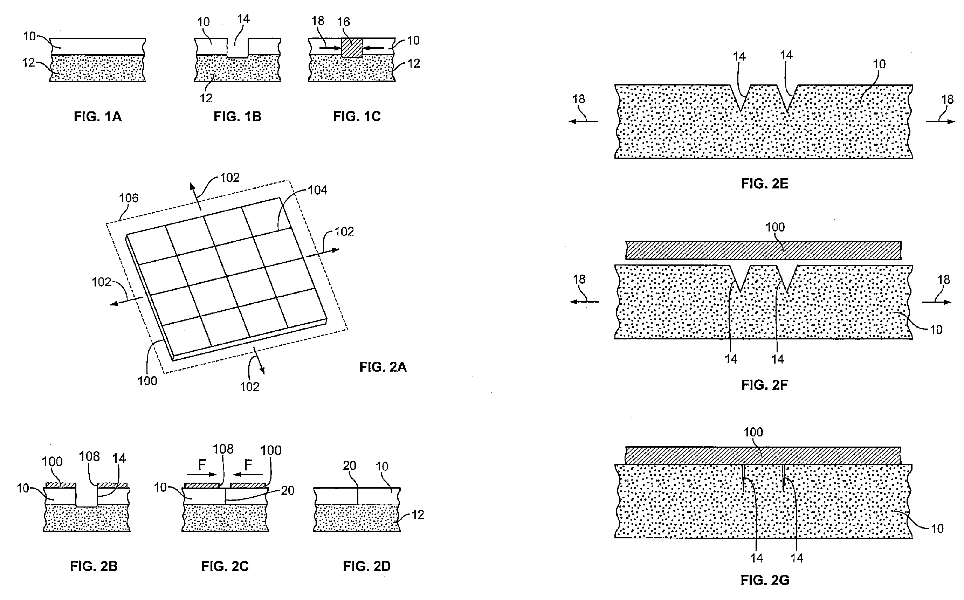

Figs. 1A to 1C illustrate the process of a conventional skin tightening procedure performed on a section of skin. As illustrated in Fig. 1A, the dermis **10** is located over a region of subcutaneous fat **12.** During a skin tightening procedure, as shown in Fig. 1B, a physician creates one or more lesions **14** (i.e., areas of treated tissue or actual openings in tissue) in the dermal region **10.** The skin tightening procedure relies on the adjacent healthy tissue to produce a heating response **16** or scar tissue that contracts the adjacent regions of the dermis layer **10** as shown by arrows **18.** The purpose of the scar tissue **16** is to create a state of traction for the adjacent region of tissue to produce a tissue tightening effect. The single lesion shown in the figures is for illustrative purposes only. Clearly, the number of lesions **14** created during the procedure will vary. Moreover, the location of the treatment depends upon the particular procedure and region of skin to be cosmetically treated.

In conventional skin tightening procedures, there is variability in the healing phase of the dermis **10.** This variability can lessen the desired cosmetic effect. The dressings described herein are intended to reduce this variability and provide an improved effect as a result of the healing process. Fig. 2A illustrates one example of a wound dressing **100** according to the present disclosure that assists in the healing process to improve the outcome of the procedure. The dressing **100** can have any number of shapes as desired by the intended application. For example, the dressing shown in Fig. 2A has a rectangular shape. However, variations include dressings **100** having contoured shapes to accommodate placement of the dressing around or near various anatomic features. Moreover, the dressing can be cut to fit to a desired anatomic region or to alter the characteristics of the dressing as described below. Alternatively, the dressing can be fit to the desired anatomic region and cut or shaped after it is placed on the region. Exemplary construction and/or material(s) used for the dressing may include those disclosed in U.S. 20110152738.
In some variations, the dressing comprises an elastic material configured with a load per width of at least 0.35 Newtons per mm at an engineering strain of 60% or a load per width of at least 0.25 Newtons per mm at an engineering strain of 45%. The elastic material may have a load per width of no greater than about 2 Newtons per mm at the engineering strain of about 45% to 60%, about 1 Newtons per mm at the engineering strain of about 45% to 60%, about 0.7 Newtons per mm at the engineering strain of about 45% to 60%, or no greater than about 0.5 Newtons per mm at the engineering strain of about 45% to 60%. The elastic material may have a load per width that does not decrease from an engineering strain of 0% to 60%, a load per width plot that increases linearly from an engineering strain of 0% to 60%, or a load per width plot that is not convex from an engineering strain of 0% to 60%. The elastic material may comprise an adhesive configured to maintain a substantially constant stress in the range of 200 kPa to about 500 kPa for at least 8 hours when strained to an engineering strain of about 20% to 30% and attached to a surface. The elastic material may comprise an adhesive configured to maintain a substantially constant stress in the range of 200 kPa to about 400 kPa for at least 8 hours when strained to an engineering strain of about 20% to 30% and attached to a surface. The substantially constant stress may vary by less than 10% over at least 8 hours, or by less than 5% over at least 8 hours.

Fig. 2A shows one variation of a dressing **100.** In this example, the dressing can comprise an elastomeric dressing (e.g. fabricated from silicone) that can optionally stretch as shown in directions **102** to expand the dressing **100** to an expanded profile **106.** The dressing device **100** can further optionally include any configuration of adhesive **104** to adhere to skin. The adhesive should not prevent the skin and/or the lesions from compressing as described herein. Alternatively, variations of dressings can allow for adhesive to be applied prior to or during application of the dressing on tissue.

The device can be placed on the dermis 10 as shown in Fig. 2B. As discussed below, the dressing **100** can be placed on the dermis **10** either prior to, during, or subsequent to creation of the lesion **14.** Once the lesion **14** is created (or during creation of the lesion **14**), the dressing **100** applies a closure force **F** to contract the lesion **14** so that the dressing **100** causes the opening **14** in tissue to close as illustrated in Fig. 2C. The tissue formed **20** as a result of the healing response then maintains the dermis **10** in a state of traction even after removal of the dressing **100** as shown in Fig. 2D. The closure force **F** can be applied in a single direction/axis or in a planar direction (e.g., X-Y axes). Stretching of the dressing **100** can be uniform or non-uniform depending upon the area of placement. As described below, variations of the dressing **100** can be configured to provide for stretching in pre-determined directions or amounts.

In those variations where the dressing **100** is elastomeric and pre-stretched, the release of the dressing **100** from the stretching force creates the closure force **F** that is applied on tissue. In alternate variations, the dressing **100** can provide a closure force via thermal, electrical, chemical or other activation of an appropriately configured dressing **100.** In such cases, the dressing applies little or no force unless activated.

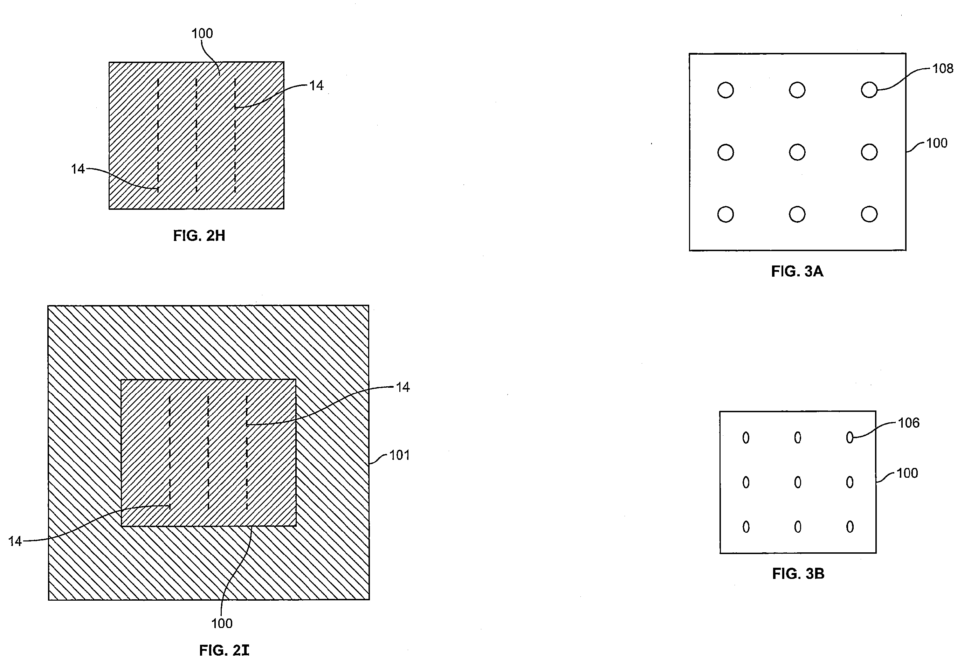

As shown above, the dressing **100** can have openings adjacent to the lesion **14.** Fig. 3A illustrates these openings **108** in a dressing **100** where the dressing is strained or expanded. The openings **108** can be pre-made in the dressing **108** or can be created during the application of energy to the tissue. As shown in Fig. 3B, the openings **108** reduce in size or can close when the dressing **100** returns to the compressed or reduced state. The openings **108** can serve an additional purpose of allowing moisture to pass through the dressing **100.** Accordingly, variations of the dressing **100** can include additional openings **108** solely allow for passing of moisture through the dressing **100.**

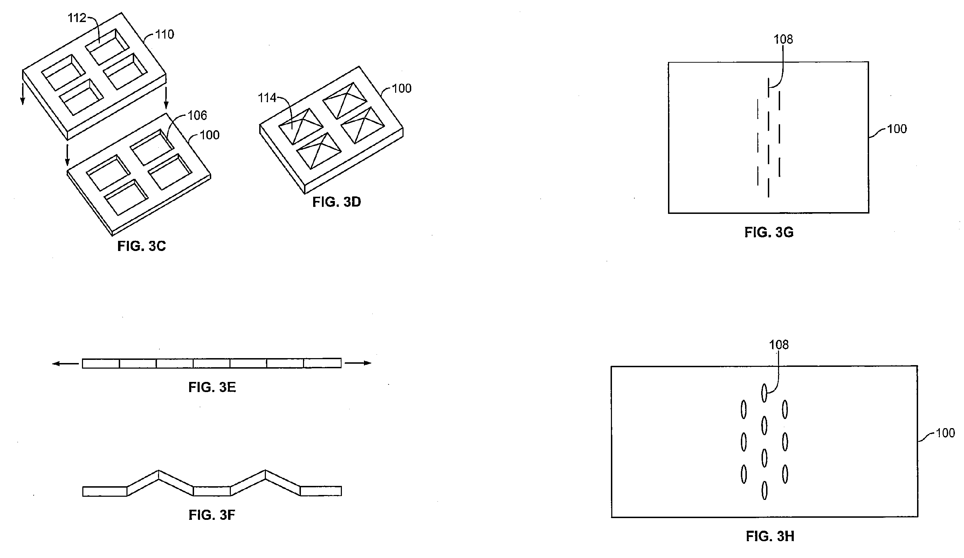

The openings may have any of a variety of shapes, including circles, ovals, triangles, square, rectangular, star-shaped, etc. Each of the In some variations, the openings (or a portion of the openings) may comprise potential openings which appear to be slits or cuts in the dressing, which then open or spread apart upon tensioning. Figs. 3G and 3H depict one example of a dressing **100** in a unstrained and strained configuration, respectively, with openings **108** comprising slits which are transversely oriented to the direction of tensioning, which form double-tapered openings upon straining. The slits or cuts may be linear or curved, isolated or branching, etc.

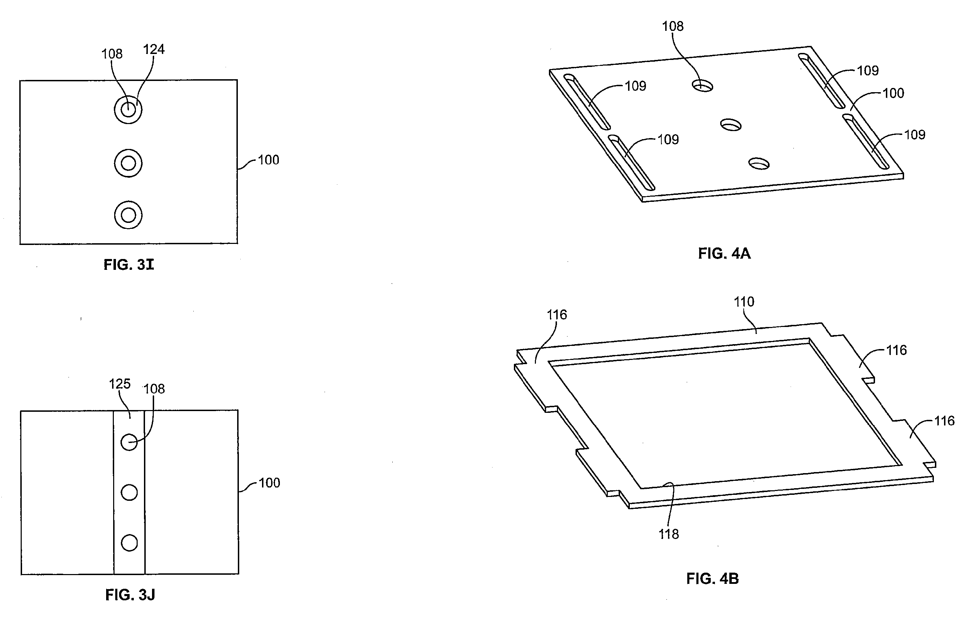

In some further variations, one or more of the openings may be reinforced with a thicker or non-elastic material to reduce or control excessive strain in the dressing material about the openings, in comparison to regions without openings. In some variations, the reinforcement may comprise open or closed perimeter, ring-like structures surrounding the openings. Materials that may be used for the reinforcement structures include but are not limited to low-density polyethylene (LDPE), fluorinated ethylene propylene (FEP) or nylon. The openings **108** of the dressing **100** may comprise individual reinforcement structures **124,** as depicted in Fig. 3I, or a regional reinforcement structure **125** that reinforced multiple openings **108,** as depicted in Fig. 3J. In other variations, the reinforcement structure may comprise a layer of material embedded or attached to a surface of the dressing, the layer of material comprising an inelastic material or a material with reduced elasticity relative to the dressing. The layer of material may comprise identical openings, slits or cuts as the dressing, or may have a different configuration. In still other variations, no openings, cuts, or slits are provided in the dressing, but indicia may be provided on the dressing to facilitate the formation of a skin lesion pattern through the dressing. In some variations, the indicia may comprise a uniform graphical grid depicted on the dressing, but in other variations, a non-uniform grid or other non-uniform pattern is provided.

Figs. 2E to 2G show another variation of application of a dressing **100** for use in the methods and devices described herein. In this variation, as shown in Fig. 2E, the tissue is placed under a state of traction or is strained as shown by application of a straining force 18 and the lesions 14 (or a single lesion) are created. Next, as shown in Fig. 2F, a physician positions a dressing **100** adjacent to or on the region of tissue **10** and over the lesions **14.** The dressing **100** can have openings over the site of the lesions **14** or the dressing **100** can be continuous. Fig. 2G shows removal of the strain from the tissue 10 which causes closure of the lesion **14.** Once the strain is removed and the tissue relaxes is the dressing adhered to the tissue **10.** Adhering of the dressing can occur via an adhesive on the dressing, activation of the dressing, or application of an adhesive between the dressing and tissue.

Figs. 2H and 2I illustrate another aspect of a use of dressings 100 that can be combined with the methods and devices described herein. In this variation, a dressing 100 is placed upon one or more lesions 14 as described herein. However, a second dressing or frame **101** is positioned to overlap the first dressing **100.**

The dressing **100** can be retained on the skin by any number of mechanisms. For example, some variations include an adhesive located between the skin and dressing. Alternate variations include the use of a tape or other sealing means placed around or at edges of the dressing **100.** The use of mechanical fasteners, e.g., staples, sutures, etc. is also within the scope of this disclosures.

Fig. 3C illustrates another variation of a dressing **100** under the present disclosure. In this variation, the dressing comprises a stretchable layer as discussed above with any number of optional window or openings **106.** Once the dressing **100** is stretched, a frame or brace **110** can be applied to the dressing **100** to maintain the dressing **100** in the stretched configuration. This may be accomplished through a fastening system (e.g., clamps or teeth) or via a temporary adhesive. The brace **110** can include any number of windows or openings to provide an unobstructed path to the tissue to create the lesions. Once the treatment occurs, the brace **110** can be removed from the dressing **110** to permit the dressing **100** to compress the lesions. In some variations, the brace **110** comprises a flexible but inelastic or incompressible material configured to resist a compression load per millimeter width of at least about 0.1 Newtons, about 0.2 Newtons, about 0.3 Newtons, about 0.4 Newtons, or about 0.5 Newtons, or more. In still other variations, the brace **110** may comprise a malleable material or a substantially inflexible or rigid material. In further variations, in the brace **110** may be pre-shaped with a generally planar shape, but in other variations, may be pre-shaped to a semi-cylindrical or other arcuate shape along one dimension of the brace **110.**

Figs. 3D to 3F show another variation of a dressing **100.** In this variation, instead of openings, the dressing **114** includes any number of raised surfaces **114.** Prior to application on tissue, this variation of the dressing **100** is stretched out, as shown in Fig. 3E. When applied to tissue the dressing 100 reverts to its pre-stretched shape so that the raised surfaces **114** separate from the surface of the tissue as shown in Fig. 3F. In such a variation, adhesive can be applied to the planar portion of the dressing **100** around the raised areas **114.**

Any of the dressings **100** shown above (and/or braces) can be mated or aligned with the lesion-creating device so that the dressing can be applied with a single device. Alternatively, a number of dressings **100** can be fitted to the treatment device in a cartridge-like form so that the physician can position a dressing onto the treatment device prior to application of the device to tissue.

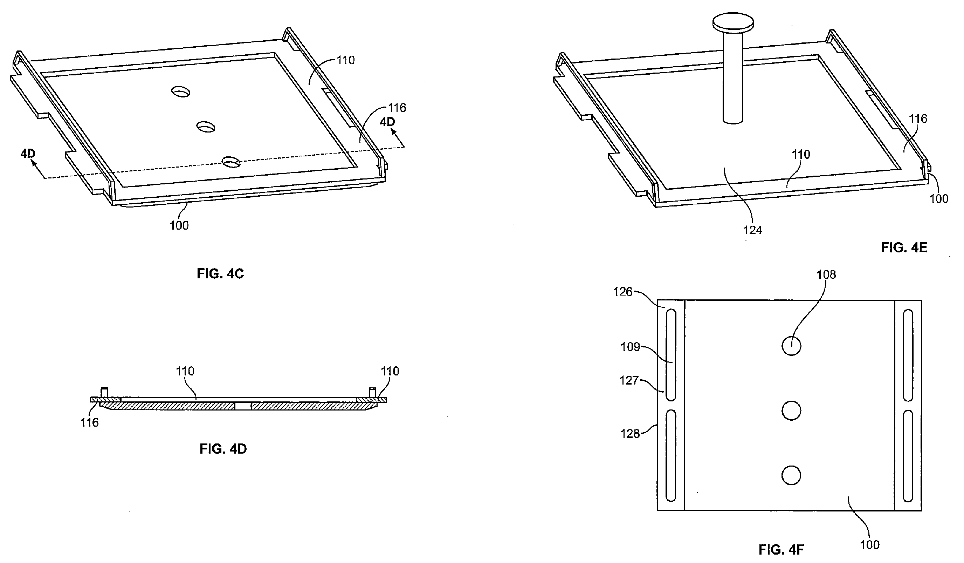

Fig. 4A illustrates another variation of a dressing **100** for use as described herein. In this variation, the dressing **100** includes any number of openings **108** that allow for creation of the lesions. In additional variations, the dressing **100** may not have any lesion-openings **108.** In such a case, the treatment could take place through the dressing **100** or the openings **108** could be created after the treatment. In any case, this dressing variation includes one or more retention openings **109.** The retention openings **109** are designed so that the dressing can be stretched over a mask or brace **110** as shown in Fig. 4B. The brace **110** will include a number of mating retention tabs **116** so that the dressing can be stretched across the brace **110** and retained on the tabs **116.** In this variation, the brace **110** includes a single central opening **118.** However, as described above, the brace **110** can include any number of openings (i.e., to match the openings on the dressing, or to provide structural rigidity to allow for stretching.) Typically, the brace **110** is stiff or inflexible when compared to the dressing **100.** However, variations can include a brace **110** that is stiff when compared to the dressing **100** but is malleable so that the brace can conform to the contours of a targeted region of tissue. As mentioned previously, the lesion-openings **108** (or other drainage or access openings of the dressing) may be individually reinforced or regionally reinforced to reduce or control any relative greater strain that may occur in regions with openings **108** in comparison to regions of the dressing **100** without opening. As depicted in Fig. 4F, the retention openings **109** of the dressing **100** may also be reinforced with a reinforcement structure **126,** which may facilitate manual stretching and attachment of the dressing **100** to the brace **110** by the user. Reinforcement of the retention openings **109** may be beneficial reducing excessive straining of the dressing region **127** between the retention opening **109** and the closest adjacent edge **128** of the dressing **100.**

Fig. 4C illustrates the dressing **110** stretched across the brace **110** with the retention tabs **116** inserted into the retention openings **109** of the dressing **100.** Fig. 4D illustrates a cross sectional view taken along the line 4D-4D from Fig. 4C. To release the dressing **100** from the brace **110,** the ends of the dressing **100** can be pulled away from the brace when the dressing engages tissue. As discussed herein, the dressing **100** can include an adhesive to secure the dressing onto tissue. Alternatively, an adhesive or other fastening means can be applied to the dressing when the physician places the dressing on tissue. Fig. 4E illustrates a variation of a dressing **100** located on a frame **116** with an applicator that can be used to secure the dressing **100** against tissue as the dressing **100** is released from the retention tabs **116.**

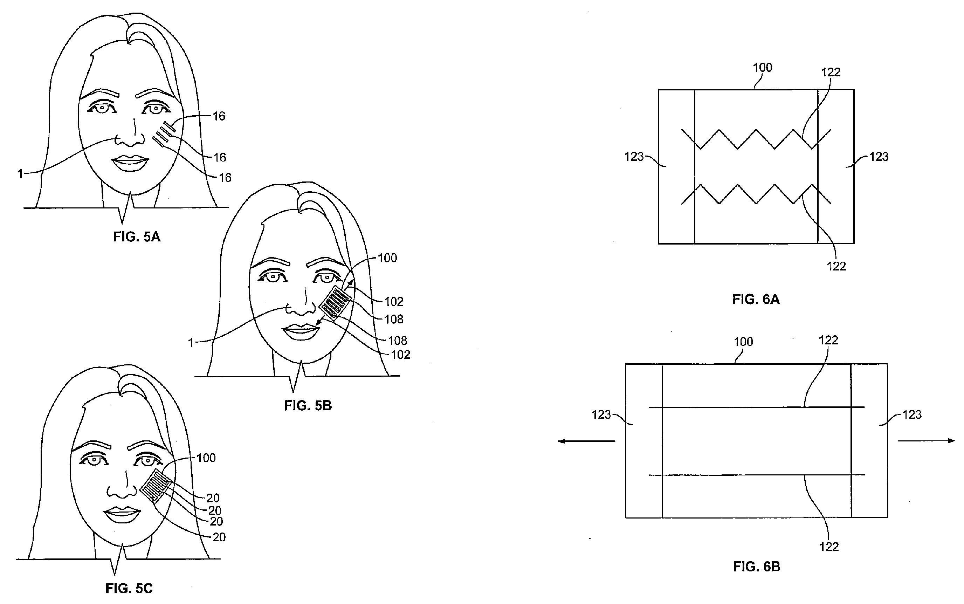

Figs. 5A to 5C illustrate another variation of a dressing **100** according to the present disclosure. In this variation, the dressing can be configured to provide a uni-axial compression (compression along one axis) where required or a unidirectional compression (i.e., movement of the dressing occurs in a pre-determined direction). Fig. 5A shows a series of lesions **16** created on a face **1** of a patient. In this example, the therapeutic procedure creates the lesions **16** in a line array type pattern as opposed to a plurality of points or dots, where the lines of lesions **16** are arranged or aligned in a pre-determined direction. For example, the pattern can be made in alignment with, against, and/or orthogonal to Langer's lines, also referred to as cleavage lines. Langer's lines are topological lines drawn on a map of the human body. These lines correspond to the natural orientation of collagen fibers in the dermis and epidermis and can be defined by the direction in which the skin of a human cadaver splits when struck with a sharp point. In practice, a series of dressing can be applied with differing orientations corresponding to typical or mapped Langer's Lines.

For example, the physician can create lesions aligned to a directional path that is similar to sutures that could be used to tighten the tissue. Fig. 5B illustrates a dressing **100** stretched in direction **102** and having a plurality of openings **108** that open along the stretched direction. As discussed above, the dressing **100** can include an adhesive for securing to tissue. Alternatively, the adhesive can be applied between the skin and dressing or another mode of fixation can be employed. Fig. 5C shows the dressing **100** upon reaching a pre-stretched or relaxed state. As shown, the openings **108** reduce in thickness to compress the lesions when forming the tissue **20** generated as a result of the healing process.

In another variation, a dressing **100** can be adhered to tissue or skin without significantly stretching and then is stretched. Stretching the tissue in this manner uniformly and gently stretches the skin underlying the dressing in a direction along Langer's lines along with the dressing.

When the skin is then treated through the dressing in a stretched condition, releasing the stretch as well as removing the dressing will allow a gentle closure force due to the skin's own natural elasticity. Presently, physicians stretch tissue using their fingers to stretch the tissue area as they treat. However, this process increases overall procedure time since the physician is only able to treat small areas at any given time. A variation of present invention includes a dressing that is stretched after being applied to the skin to stretch the skin along Langer's lines. Once the tissue is treated, the dressing is removed. This permits the natural resiliency of the skin to provide a gentle closure force without having to leave the dressing in place.

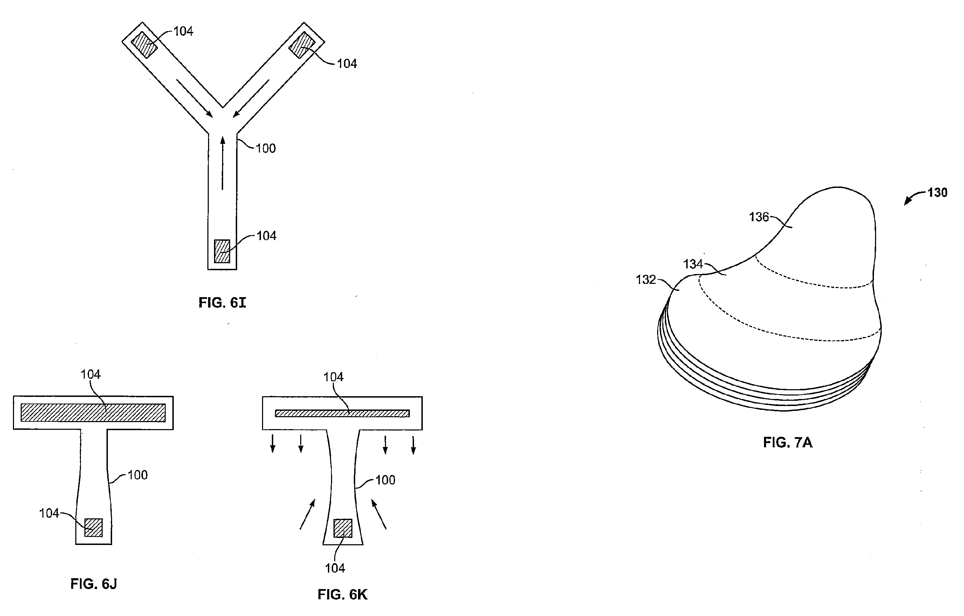

Figs. 6A and 6B illustrate an aspect of dressings **100** described herein.

In this example, the dressing **100** includes one or more limiting members **122** to predetermine a strain capacity of the dressing **100.** For example, as shown in Fig. 6A, the limiting members **122** are shown in a compressed configuration. When the dressing is stretched, as shown in Fig. 6B, the limiting members **122** approach their predetermined length to limit the maximum strain of the dressing **100.** Accordingly, a physician can be provided with a number of dressings **100** each having a varying amount of maximum strain. Although Figs. 6A and 6B illustrate a dressing having uni-directional strain or deflection, variations of the invention contemplate limiting members that limit strain in a bi-axial direction. The limiting members **122** can be comprised of a shape memory alloy such as a super-elastic alloy or a heat activated alloy that extends or contracts in response to a temperature shift. Although a zig-zag shape is depicted for the limiting member **122** in Fig. 6A, in other variations, the limiting member may comprise an undulating configuration or other curved configuration, or any non-linear configuration. The limiting member may comprise any of a variety of relaxed threads, strings, wires or other elongate, elongatable, straightenable or stretchable members that straighten, lengthen and/or stretch to a desired amount, degree, and/or preset limit. The limiting members may be configured with a sufficient tensile strength to prevent, resist or otherwise control over-stretching of the dressing. The skin treatment device may be constructed of multiple layers of an elastic material such as silicone with an adhesive between layers to which the members are attached initially in the relaxed, undulating, sinusoidal, unstraightened or other unstretched configuration. According to a variation, the shape limiting strings or other devices may prevent straining in regions where straining or less strain is desired. For example, the threads may be straight at the edges of the skin treatment device to prevent straining at the edges. In some variations with multiple limiting members, the limiting members may be equally spaced apart and have uniform lengths and uniform attachment points across a transverse dimension to the tensioning axis of the dressing. In other variations, the limiting members may have a variable or non-uniform spacing, may have non-uniform lengths, non-uniform attachment points, and may also be serially arranged along the tensioning axis. The opposing ends of the limiting members **122** may also optionally have removable (or non-removable) manipulation elements **123** attached to the ends to provide for a more even or uniform strain, and may comprise an inelastic material which is the same or different as the limiting members **122.** The manipulation elements **123** may span the entire transverse dimension of the device 100 to the axis of tensioning, as depicted in Figs. 6A and 6B, but in other variations may be less than the entire transverse dimension (e.g. having a transverse dimension sufficient to span a plurality of limiting member **122,** but less than the full transverse dimension of the dressing **100.** Such elements may comprise planar members, handle members, flexible members and/or inflexible members. They may be attached and removed in a variety of manners, for example as described herein.

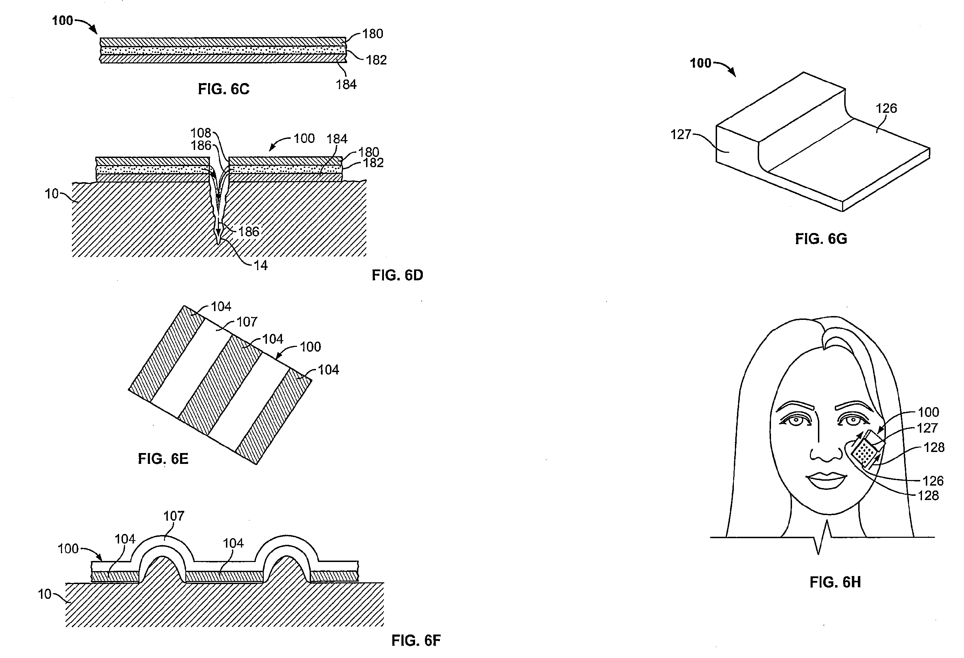

Fig. 6C illustrates another variation of a dressing **100** that incorporates a bio-active substance that is intended for delivery to or near the site of the lesion. In one example, the dressing **100** includes a polymer layer (e.g., silicone) and an adhesive layer **184.** The dressing **100** can also include a bio-active substance **182.** Although the bio-active substance is shown as a separate layer **182,** variations of the dressings can include a bio-active substance that is infused with the polymer layer **180** and/or adhesive layer **184.** Alternatively, the bio-active substance can be a separate layer. Fig. 6D illustrates the dressing **100** of Fig. 6C after an opening **108** is made in the dressing **100** during creation of a lesion **14.** As shown, creation of the opening causes the bio-active substance to enter the lesion **14** as represented by arrows **186.** The bioactive substance can be an activated substance, such as a Rose Bengal dye (a photosensitive dye typically used to cross-link collagen and is activated by light having wavelengths of 514 nm, 532 nm or 458 nm). Alternatively, the bioactive substance can be any drug or pharmaceutical substance delivered for a particular effect on the lesion or tissue. In further examples, the substance can be a material that causes cross-linking of collagen, such as riboflavin and/or glucose. Examples of other bio-active agents that may be used include hemostatic or coagulative agents to help reduce bleeding. Such agents include chitosan, calcium-loaded zeolite, microfibrillar collagen, cellulose, anhydrous aluminum sulfate, silver nitrate, potassium alum, titanium oxide, fibrinogen, epinephrine, calcium alginate, poly-N-acetyl glucosamine, thrombin, coagulation factor(s) (e.g. II, VII, VII, X, XIII, Von Willebrand factor), procoagulants (e.g. propyl gallate), antifibrinolytics (e.g. epsilon aminocaproic acid), and the like. In some variations, the agents may be freeze-dried and integrated into the dressing and activated upon contact with blood or other fluid. In some further variations, an activating agent may be applied to the dressing or the treatment site before the dressing is used on the subject. In still other examples, the hemostatic agent may be applied separately and directly to the wound before application of the dressing, or after application to the dressing via a catheter or tube. The devices may also comprise one or more other active agents that may be useful in aiding in some aspect of the wound healing process. For example, the active agent may be a pharmaceutical compound, a protein (e.g., a growth factor), a vitamin (e.g., vitamin E), or combinations thereof. A further example of such medicament may include, but is not limited to various antibiotics (including but not limited to cephalosporins, bactitracin, polyxyxin B sulfate, neomycin, polysporin), antiseptics (such as iodine solutions, silver sulfadiazine, chlorhexidine), antifungals (such as nystatin), antiproliferative agents (sirolimus, tacrolimus, zotarolimus, biolimus, paclitaxel), grow factors (such as VEGF) and other treatments (e.g. botulism toxin. Of course, the devices may comprise more than one medicament or agent, and the devices may deliver one or more medicaments or agents.

Fig. 6E and 6F show another variation of a dressing 100 having an adhesive pattern 104 that varies on the dressing 100 as opposed to being located on the entire surface of the dressing 100. Fig. 6E shows one example of a varying adhesive pattern 104 where the adhesive layers alternate with uncovered sections 107. When applied to tissue 10 and released from the strained condition, the dressing 100 contracts at the uncovered regions 107 causing the tissue 10 to buckle at the uncovered regions 107 as shown in Fig. 6F. Other examples of variable adhesive patterns on the dressing are described in U.S. Pub. No. 2011/0152738, filed on August 11,2010.

Fig. 6G shows another variation of a dressing **100** for use as described herein. In this variation, the dressing comprises a varying elasticity across different sections. For example, as shown, a dressing can have an elastic section **126** coupled to a relatively less-elastic section **127.** The illustrated variation shows the elastic section **126** as being thinner than the less-elastic section **127.** However, any number of configurations can provide varying stretch or elasticity. For example, the different sections can have different reinforcements, different materials with varying durometers. One such use of the varying dressing **100** is shown in Fig. 6H, which shows the less elastic section **127** placed in an area where stretching of the skin is not desired and the elastic section **126** being used to produce a pulling force in a desired direction as shown by arrows **128.** Other examples of dressings with variable elasticity regions are described in U.S. Pub. No. 2011/0152738. In one particular example, a dressing may be tapered near its edges to reduce thickness. A tapered edge may also ameliorate peak tensile forces acting on skin tissue adjacent to the adhesive edges of the dressing. This may or may not reduce the risk of skin blistering or other tension-related skin trauma. In other variations, the edges of the dressing may be thicker than the middle of the dressing. It is hypothesized that in some configurations, a thicker dressing edge may provide a relative inward shift of the location of the peak tensile forces acting near the dressing edge, compared to dressings of uniform thickness.

Figs. 6I to 6K show additional variations of dressings **100** that provide directional or vectored application of force. As shown, the dressings **100** can include adhesive **104** such that when applied to tissue the dressing is pulled to provide a strain in tissue in a desired direction. Benefits of such a variation include providing the physician with control over the degree of strain. Furthermore, the dressings can be treated with, for example, a chromophore to monitor the degree of strain in the dressing. Other examples of a color change material or structure that may be used or incorporated into the dressing are described in U.S. Pub. No. 2006/0246802 to Hughes et al.

Fig. 7A illustrates another variation of a dressing **130** that includes a contoured shape for suited for placement on specific anatomy (for example, the illustrated dressing **130** can be placed on a face of a patient having a larger area at a lower region to accommodate placement along the jaw-line and a smaller surface area at a top region to accommodate placement close to the ear). Fig. 7A also illustrates the dressing **130** as having a varying thickness across the length of the dressing **130.** The varying thickness allows for a physician to adjust the amount of strain that the dressing **130** applies to tissue. For example, section **132** can include the greatest number of layers, which translates into the highest strain rate. To reduce the strain rate, the physician or medical practitioner would cut or remove section **132** from the dressing **130.** Removing section **132** leaves sections having fewer layers and as a result, lower strain rates. Although the figure illustrates three sections **132, 134, 136,** any number of sections is within the scope of this disclosure.

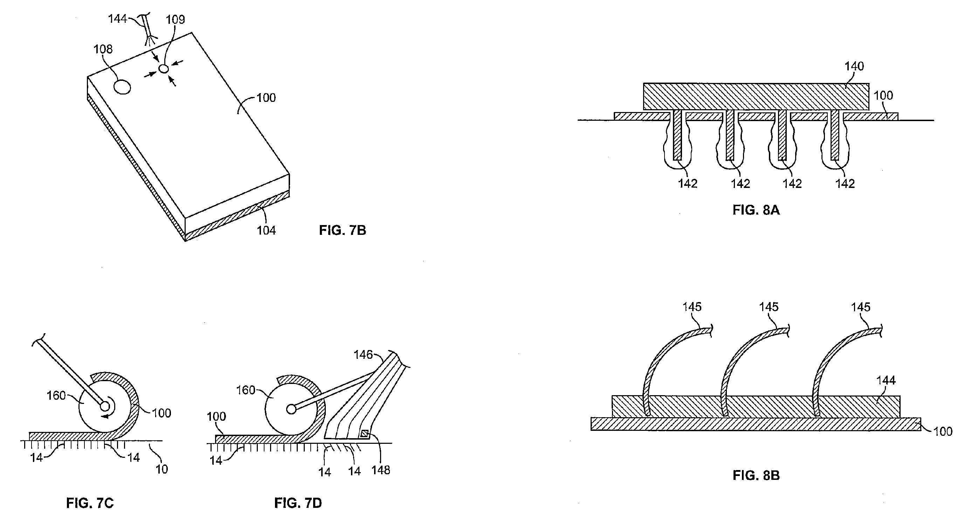

Fig. 7B illustrates a further variation of a dressing **100** that includes a thermally responsive material. In this variation the dressing **100** can include one or more openings **108** but the construction of the dressing **100** allows for customization of the location as well as degree of strain that the dressing **100** will apply on tissue. The dressing can be adjusted using visible or UV light, or it can be activated using a chemical response or via heat. The dressing can also include a separate layer that drives shrinkage of the dressing upon activation (e.g., via a laser, heat, other irradiation). In any case, use of a shrinkable dressing permits customization to create tension or strain in a desired direction during the treatment procedure.

Figs. 7C and 7D illustrate another mechanism for application of a dressing **100.** As shown, a dressing **100** can be applied using a roller applicator **160.** The roller applicator **160** can strain the dressing **100** as it applies the dressing **100** to tissue **10** over the lesions **14.** Alternatively, the roller applicator **160** can apply a pre-strained dressing **100** that compresses tissue **10** and the lesions **14** upon positioning on the tissue **10.** Fig. 7D illustrates a roller applicator **160** that is coupled to a lesion creating device **146,** which creates the lesion **14** and where movement of the device **146** causes the roller **160** to apply the dressing **100.** The device **146** can also include one or more sensors **148** to provide feedback of location to the system or can be used to detect movement of the device **146.** In some variations, the roller applicator **160** may comprise a ribbon spring (or other spring mechanism or tension control mechanism) to resist rotation of the roller until a specific tension threshold is achieved.

Fig. 8A shows another application of a dressing **100** as described herein. In this variation, the dressing **100** is coupled to a treatment device **140.** The treatment device **140** can create a lesion through any variety of treatment modes where the active elements **142** of the treatment members pass through openings in the dressing. For example, the treatment device **140** can create the therapeutic lesion using RF energy, plasma, cryogenic energy, microwave energy, laser, optical energy (non-laser) chemical, resistive heat, ultrasound energy, or via mechanical energy. As shown, a dressing **100** can be coupled to the treatment device **140** so that after creation of the lesions, the treatment device **140** can be removed leaving the dressing in place to apply compression to the lesions. Alternatively, the treatment device **140** can be directly applied over a dressing **100** that was previously positioned. In some variations, the dressing **100** may comprise a pattern of treatment openings with predetermined size, spacing or location, and the treatment device **140** may comprise a matched pattern or array of treatment elements **142** with the same spacing or location. In some variations, the pattern of treatment openings in the dressing **100** may comprise a repeating sub-pattern, and the treatment device **140** may comprise a matched pattern or array of treatment elements **142** to the dressing subpattern of openings. In these variations, the dressing **100** may comprise indicia to delineate the locations of the subpatterns. Thus, the treatment device **140** may be used at multiple regions of the dressing **100.** The pattern or array of treatment elements **142** of the device **140** may comprise at least 2, 3, 4, 5, 6, 7, 8, 9, 10, 15, or 20 treatment elements.

Fig. 8B illustrates another variation of a dressing **100** in combination with a treatment device **144.** In this variation, the treatment device **144** creates openings within the dressing **100** before or during the actual treatment. For example, the treatment device can employ a laser, optical/light, chemical, ultrasound or electrical energy to create an opening through the dressing while creating the therapeutic lesion in tissue beneath the dressing. Moreover, device **144** can apply energy to a dressing **100** to activate the dressing **100** induce or adjust strain. In such a case, the dressing can be in an unstrained condition prior to activation by the device **144.** Alternatively, or in combination, the device **144** can reduce or increase a strain in the dressing depending upon the desired application. Fig. 8B illustrates lesion creating elements **145** positioned adjacent to the dressing **100.** In the event that the device **144** employs a laser to create lesions, the laser is emitted from the elements **145** and can either pass through the dressing **100** or can create openings in the dressing. In those variations where the lesion creating elements **145** comprise electrodes or mechanical members, the elements **145** can pass through the dressing when advanced from the device **144.**

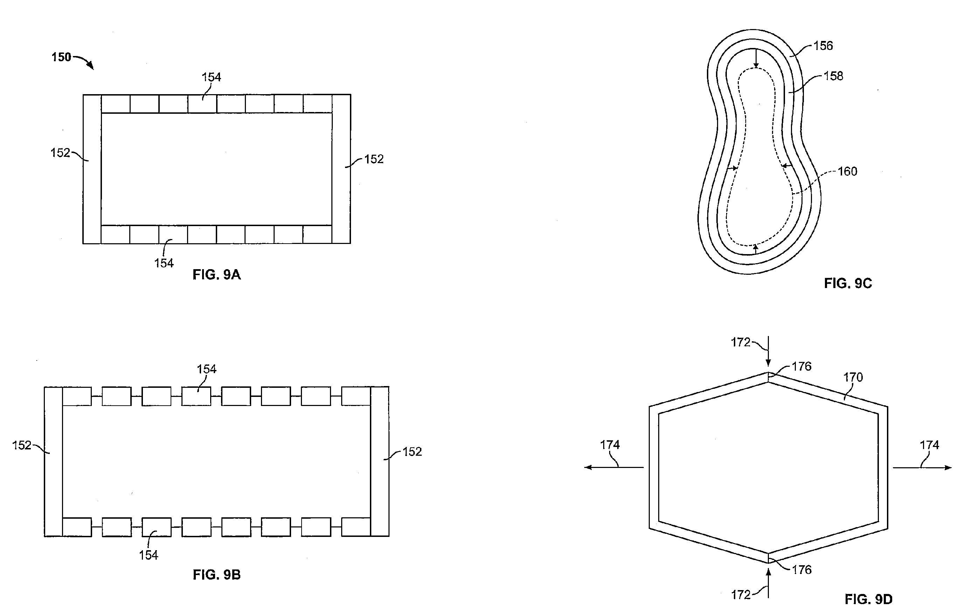

In many cases, the dressings described herein are intended for positioning on contoured tissue surfaces rather than planar surface. Accordingly, there may be a need to provide a dressing that can approximate the contoured shape prior to affixing to the tissue surface. Figs. 9A and 9B show one possible solution to adjust a contour of a dressing described herein. Fig. 9A shows a variation of an adjustable frame **150.** In this example, the frame **150** includes a plurality of adjustable links **154** coupled between two end-pieces **152.** However, variations of the frame **150** include adjustable links **154** forming the entire periphery of the frame. As discussed above, the frame **150** can include one or more fixtures to permit retention of the dressing. Fig. 9B illustrates the adjustable frame **150** where one or more of the plurality of links **154** can be adjusted or extended to permit a portion or all of the frame **150** to form a contoured shape. Although not illustrated, a dressing attached to the frame **150** will then be in a better configuration when being secured to a contoured tissue surface.

In additional variations, a frame can be used without a dressing to apply strain to the tissue in a similar manner as a dressing. For example, a deformable frame can apply a compression force to the tissue and then can be affixed to the tissue in a manner as described herein (e.g., adhesive, mechanical fasteners, sutures, etc.) Doing so allows the frame itself to compress tissue to assist healing of the treated region.

Fig. 9C shows another variation of a frame **156.** In this variation, the frame **156** employs an expandable member or bladder **158.** When affixed to tissue the bladder can be inflated or expanded to position **160** to provide compression within the area bounded by the frame **156** and bladder **158.** In other variations, a foam (open cell or closed cell) may be provide within the bladder to maintain the bladder in an expanded state, and negative pressure may be applied to reduce the bladder/foam size. In still other variations, the frame may comprise a foam member in lieu of a bladder member to provide a resilient or contouring surface or structure for the frame.

Fig. 9D illustrates another example of an adjustable frame that is used to compress tissue. In this example, the frame **170** can have hinges or joints **176** that transmit a force **172** applied at the joints to result in an expansion force **174** at the edges of the frame. In some variations, the frame is biased to return to its initial shape upon application of a force. In such a case, after force **172** is applied to the frame, the frame **170** provides compression within the area bounded by the perimeter of the frame **170.** In use, a physician could apply the activation force **172** to the frame, then adhere portions of the frame **170** to tissue. As the frame **170** attempts to return to its initial shape, the return force compresses tissue within the frame. In an alternate variation, the frame **170** is not biased. Instead, the frame **170** can be affixed to an elastic dressing (not shown) as described above. In this example, application of force **172** causes expansion or straining of the elastic dressing in direction **174.** A physician or medical practitioner can then affix the strained dressing **170** to tissue where the resiliency of the tissue compresses the tissue.

The dressings of the present invention can also provide temporary results to simulate a clinical effect. These temporary results can allow a patient or physician to determine the type or amount of treatment desired. For example, a physician can position pre-strained dressings on a patient to show the patient the results of a procedure given a pre-determined amount of shrinkage or lift. Such a feature allows a physician to position dressings having a pre-determined amount of strain on a patient so that the patient can visually see the results of the given reduction. For example, a physician can position dressings that lift the skin by a given amount so that the patient can determine whether more or less lift is desired. The goal is to simulate clinical results and allow a patient to see a real time simulated clinical result via the application of the dressings. Such pre-strained dressings can be provided as a kit having varying ranges of displacement with corresponding templates to assist the physician in applying apply therapeutic treatments to match the temporary state of the tissue. In this way, a patient can observe the simulated clinical result, once a desired result is achieved; the physician can select treatment templates based on the dressings that are used to produce the temporary effect. Use of the dressings to simulate a clinical effect can be used in any number of cosmetic procedures outside of skin tightening. In additional variations, the simulated clinical effect can be used to establish a treatment plan. Such a treatment plan can include the amount of or location where the therapeutic treatment. The simulated clinical effect can be processed through a computer analysis to provide the physician with a treatment plan based on the type of dressing used or amount of lift used to produce acceptable or desired results.

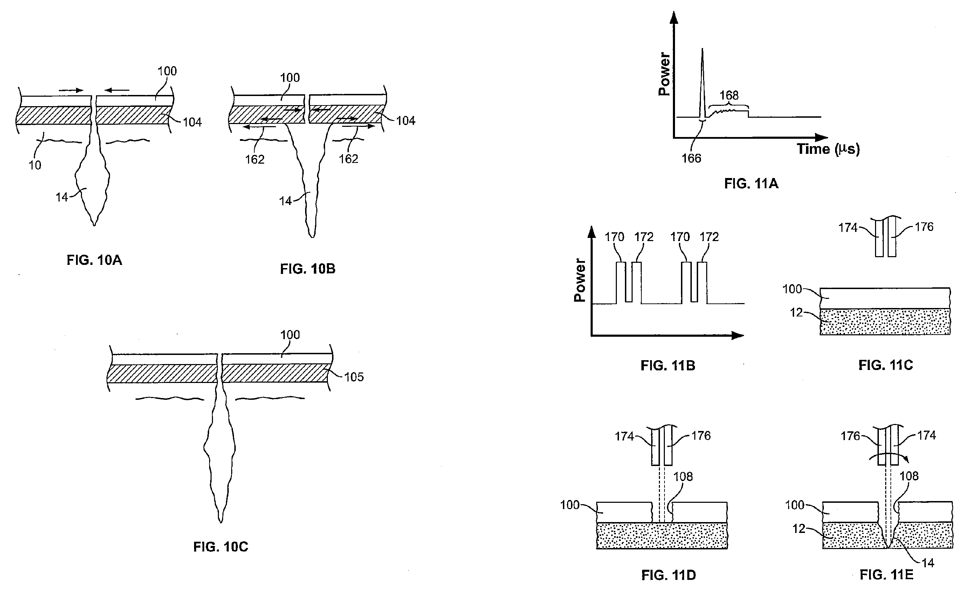

Figs. 10A to 10C illustrate another aspect for use with dressings of the present invention. As shown in Fig. 10A, a dressing **100** that is placed over a lesion **14** when used to apply compression to the dermal layer **10** of skin. In this variation, the dressing **100** includes an adhesive layer **104.** In some variations, the adhesive layer **104** is sufficient to prevent creeping or movement of the tissue **10** subsequent to placement of the dressing **100.** However, in certain circumstances, as shown in Fig. 10B, the tissue **10** contacting the adhesive layer **104** can begin to creep causing the lesion to move in the direction shown by arrows **162** resulting in opening of the lesion **14.** The creep of the tissue **10** can occur due to the restoring force of the skin, which may be higher in a particular target location. Alternatively, creeping of tissue can occur due to the thickness of the elastic layer or when the adhesive layer is non-rigid and/or deformable and takes time to secure the dressing to the tissue.

Fig. 10C provides a representation of an alternate variation of a dressing **100** having an adhesive material **105** that can be activated or set. In some variations the settable adhesive material **105** is set from a liquid or viscous phase to a solid phase via UV or IR irradiation or even oxidative curing. In some variations, use of oxidative curing allows for securing of the dressing without heating of the tissue and/or dressing. As shown in Fig. 10C, the adhesive material **105** solidifies to keep the lesion closed but also remains pliable to allow the dressing **100** to remain pliable and conform to the curvature of the tissue.

In additional variations a dressing can be used to limit the lesion size or to minimize collateral damage to tissue. For example, a solid dressing can be applied to tissue as described above. Next, a laser can then be used to create openings in the dressing as well as to create the lesion. For instance, an Er:YAG laser or an Er:YsGG may be used with a Cr2+:or CR2t: ZnSe Q-switching device, or a Cr2+:Cr2t:ZnS Q-switching device to apply a pulse in a Q switched mode followed by a free running mode or normal mode. The pulse could be used to remove a portion of the dressing since the pulse produces a plasma initiation and expansion to photomechanically remove the layer of the dressing (e.g., a silicone layer). The free running mode operation creates a micro lesion via photothermal ablation of the tissue.

Fig. 11A illustrates an exemplary plot of power versus time for a laser treatment application so that a single laser can create an opening in a dressing and a subsequent lesion at the site of the opening.. As shown, the first applied pulse **166** reaches a power level much greater than that of the subsequent free running mode pulse **168.** One benefit of creating openings in the dressing simultaneously or immediately prior to creating the lesions is to eliminate alignment of the openings and lesions.

Figs. 11B and 11C illustrate another variation of using a solid dressing and creating openings in the dressing once the dressing contacts the tissue. As illustrated in Fig. 11C, the treatment can include two or more different types of lasers **174,** 176, where one of the lasers **174** can be selected such that it is highly absorbed by the dressing **10.** While the second laser **176** can be selected such that it is highly absorbed in tissue. For example, a C02 and Er:YAG laser can be used together. The wavelength of the CO2 laser is highly absorbed by silicone and the wavelength of the Er:YAG laser is highly absorbed in skin. In one example, the wavelength required to create openings in the dressing is 10.6 µm while the wavelength for creating lesions could range from 1.9 to 3.3 µm. The lasers can be arranged so that they are either co-axial or confocal (e.g., the laser elements if Fig. 11C can be rotated or moved so that each laser targets the same region.) Alternatively, the lasers can be configured to deliver light in a concentric manner. In use, the lasers are time-delayed as shown in Fig. 11B, which illustrates a graph of power versus time. As shown, the first laser **174** delivers a first pulse **170** to create the opening in the dressing **10.** The pulse **172** from the second laser **176** can be staggered or delayed to allow for ejection and/or removal of debris. While any variation of lasers can be used in this configuration, one desirable variation can include the use of two low power lasers to provide each source of light. Alternatively, a more complicated dual wavelength system can be used. Such a system could deliver multiple wavelengths of light as described above.

Fig. 11D shows the system of Fig. 11C, where the first laser **174** creates an opening **108** in the dressing **100** but not the skin since the laser is not absorbed by the skin. Fig. 11E shows the second laser **174** creating the lesion **14** through the previously created opening **14.**

The above system or configuration allows for a skin tightening therapy with an adhesive patch applied to skin with the skin and patch ablated in situ. The patch is applied to untreated skin under tension with an adhesive. The patch initially does not have holes over the areas where lesions are to be formed. The entire patch may be solid or may have holes or other features to position and/or stretch the patch. An initial laser beam ablates holes or other features in the patch to expose certain areas of the skin. The exposed areas can then be treated through holes in the patch. The laser parameters are such that the patch material is cleanly ablated with a minimum of thermal damage to the underlying skin. A second laser beam of possibly different character is then used to ablate a controlled amount of skin. An additional benefit of creating openings in the dressing and subsequently creating lesions is that the lesion pattern and/or openings in the dressing can be customized during a procedure rather than needing to follow the pattern of a pre-configured dressing.

One benefit of creating openings in the dressing in situ is that there is no need for orienting a dressing having pre-made openings with previously created lesions. Yet another benefit of creating openings in-situ is that the pattern or features of the holes in the dressing are customized to match the lesions.

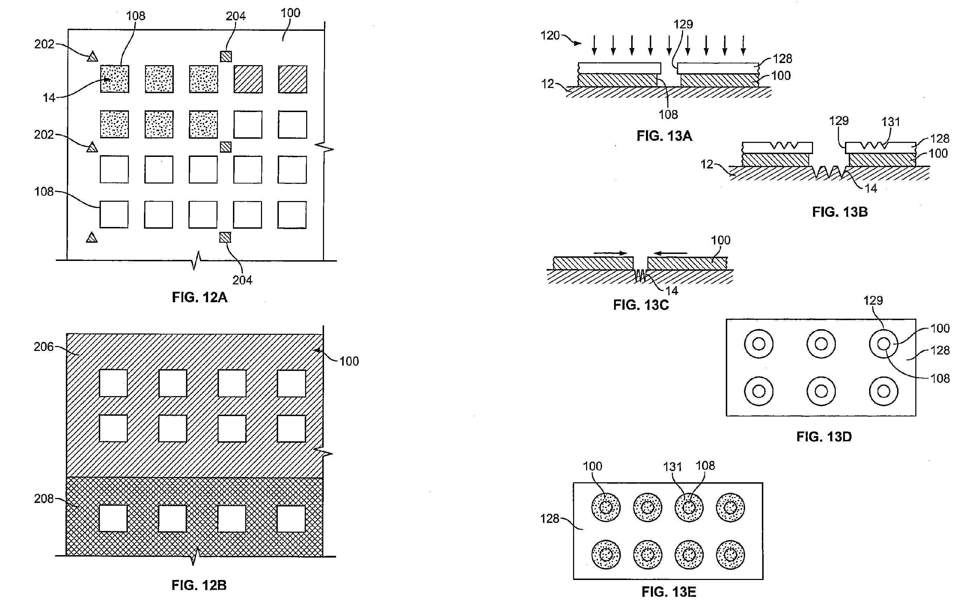

Fig. 12A shows an example of a dressing **100** having one or more registration features **202, 204** allowing for detection and/or recognition regarding characteristics of the dressing. For example, Fig. 12A shows registration features **202** and **204,** which allow for optical scanning or reflecting light that can be used to confirm alignment of the treatment device. Alternatively, or in combination, the presence or relative position of the registration features **202** and **204** can provide any range of information regarding the pattern of openings **108** or even the location where treatment should occur. For example, in Fig. 12A, lesions are created in the openings that are within specific registration features **202** and **204**

Once the system obtains feedback regarding the registration features, the system can confirm that the treatment device is in the correct position. Subsequently, image analysis hardware and software can be used to recognize the patterns or openings **108** in the dressing **100** to either control the treatment parameters, to maintain a database of treatment areas, or to perform some other custom treatment depending upon type of dressing or orientation of the dressing.

One approach is to position a handheld scanning handpiece directed at the approximate location of the treatment. The system can determine whether positioning of the handpiece is within an acceptable tolerance using an optical detection of one or more registration features **202, 204.** Once the system confirms correct or acceptable positioning, the system can then permit or trigger a treatment cycle. For example, a laser scan pattern can be released on a time scale sufficiently rapid enough (<50 msec) such that inadvertent motion of the handheld scanning handpiece does not appreciably affect the pattern. The handheld scanning handpiece can be repositioned to treat the next zone, and the optical detection of registration features at the correction positioning of the dressing with respect to the handpiece will again trigger the release of a laser scan pattern.

Alternatively, a handpiece with either scanning patterns or fixed patterns (including a single shot) can be manually positioned laterally to the dressing, with optical detection of the registration features **202, 204** triggering individual firing events. One example involves the use of an optical mouse-type arrangement in which a light source such as an LED projects onto the dressing surface and the reflected or scattered light from the dressing is re-imaged into an optical detector as the handheld device is manually moved laterally across the dressing.

Alternatively, a complete image may be acquired by an imaging system, and image-processing software may be used to create a custom scan pattern to be delivered. The positioning of the dressing with respect to the laser delivery system, the particular configuration of the dressing, the desired pattern of lesions may all be processed to determine a customized laser scanning pattern appropriate for a particular dressing and patient. In this realization, a relatively large area may be treated by a scanned laser pattern, which may require a duration that is long enough that movements of the patient with respect to the laser delivery device may occur. The invention may interrupt delivery of laser pulses is motion of the registration features 202, 204 is detected. The positioning may be restored and laser firing resumed. Alternatively, a new image may be acquired and a new laser delivery pattern computed and delivered.

Fig. 12B provides a partial illustration of another variation of a dressing **100.** In this variation, a dressing **100** can include a surface that provides a particular optical or other identifiable property that can be recognized by the treatment device to alter treatment parameters or lesion location based upon pre-determined parameters. For example, the dressing **100** can include a particular, reflective, absorptive, or scattering characteristic that is recognized by the treatment device. In addition, as shown, a dressing can include a first region **206** having a first set of characteristics, and a second region **208** having a second set of characteristics.

Figs. 13A to 13E show another variation of a dressing **100** covered by a mask **128** where the dressing **100** and mask **128** allow for a treatment device to create lesions at specific areas, thereby eliminating the need to align the dressing with the lesion. As shown, the mask **128** can include openings **129** that are in alignment with opening **108** in the dressing. Optionally, the mask **128** can apply strain to the dressing **100.** Fig. 13A illustrates the dressing **100** in a strained configuration to place the tissue **12** in a state of traction. The dressing **100** and mask **128** are exposed to the treatment energy **120** (e.g., a laser or other treatment modality). The therapeutic energy **120** creates ablation patterns **14** and **131** in the tissue **12** and the mask **128** respectively. Fig. 13C shows removal of the mask **128** and eventual compression/closure of the lesions **14** as the dressing **100** contracts. Figs. 13D and 13E illustrate top planer view of a dressing **100** and mask **128** having respective openings **108** and **129.** Fig. 13D shows the dressing **100** and mask **128** pre-treatment. Fig. 13E shows the dressing **100** and mask **128** after the ablation process. As shown, the ablation pattern **14** and **131** can be created over a wider area than the opening **14** in the dressing **108** but only forms lesions **14** in the tissue in a limited manner at the exposed areas.

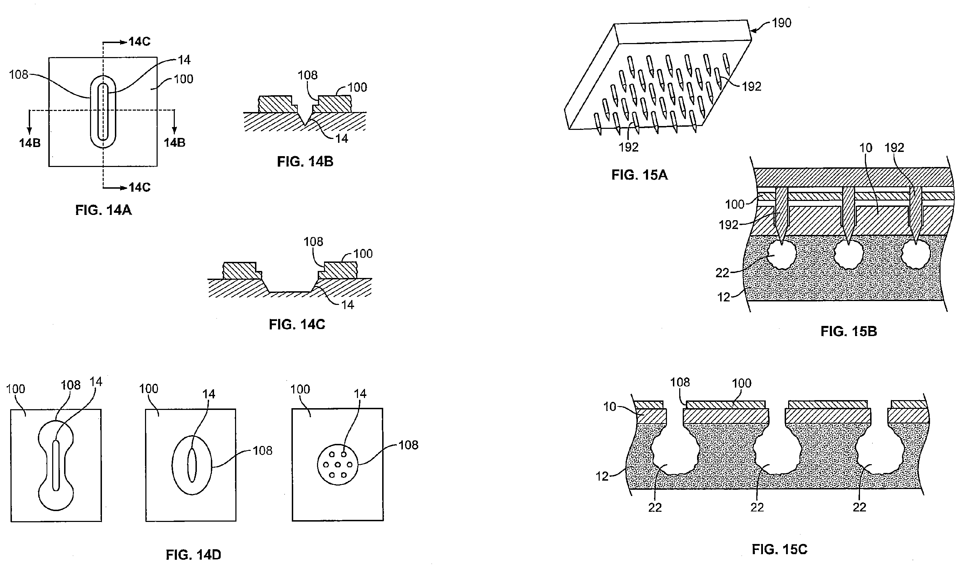

Fig. 14A illustrates another advantage of creating a lesion **14** in situ with the dressing **100.** As shown in Fig. 14A, the lesion **14** as well as the opening **108** in the dressing **100** can be made to have a unique shape that can be made or modified during the procedure. For example, Fig. 14A shows a dressing **100** with a lesion **14** in tissue, where both the lesion **14** and opening **108** are made to have a longer dimension when viewed along 14C-14C (as shown in Fig. 14C), when compared to direction 14-B-14B (and as shown in Fig. 14D). Furthermore, the lesion pattern can be a non-linear design. An additional example of such patterns is shown in Fig. 14D

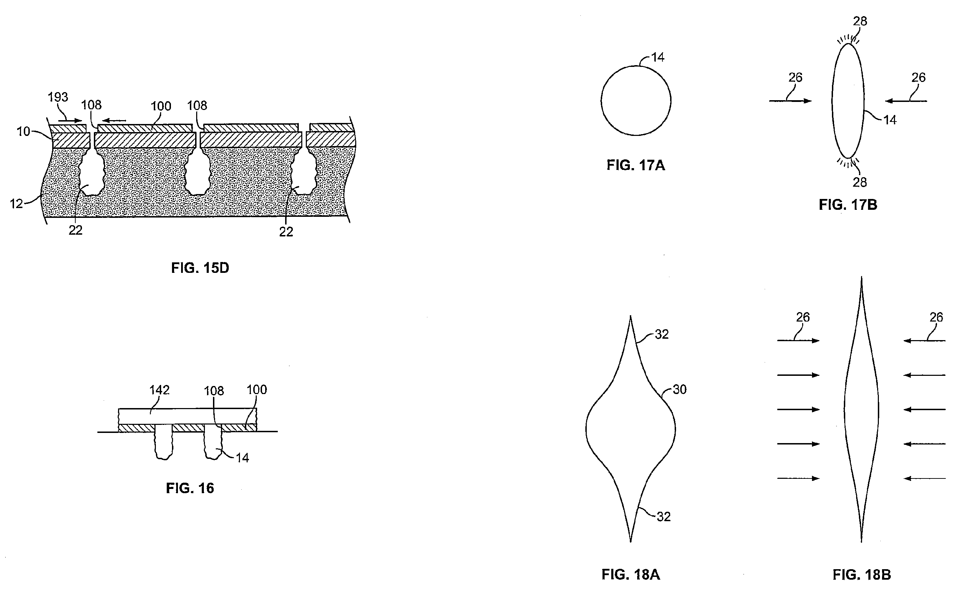

Figs. 15A to 15D illustrate another use for dressings of the present disclosure. Fig. 15A illustrates an array **190** of electrodes **192** adapted to penetrate subdermal tissue, such as subcutaneous layers **12.** Fig. 15B illustrates a dressing **100** as described herein, where the dressing is strained and placed over the electrodes **192** as shown in Fig. 15B. The electrodes **192** (e.g., RF electrodes or any other energy modality described herein) applies energy to the subcutaneous fat layer **12** to lyse, burn, or otherwise breakdown the fat to create a lesion **22** or cavity **22.** Fig. 15C illustrates removal of the electrode array **190** leaving the dressing **100** in place against the dermal region **10** of tissue. Because the dressing **100** is in a strained configuration, removal of the dressing **100** from the electrode array causes movement of the dressing **100** to close the openings **108** as described above.

Fig. 16 depicts an additional use of a dressing **100** as a mask to direct creation of the lesion **14.** As illustrated, an electrode **142** or other lesion creation element is placed against a dressing **100** such that activation of the element **142** creates lesions **14** through openings **108** in the dressing **100.** As discussed above, creating lesions **14** through openings **108** in the dressing **100** eliminates the need to match openings in the dressing to lesions. The use of the dressing **100** as a mask type element eliminates the need for electrodes penetrating into tissue.

Elastomeric dressings of the present disclosure can also be used by affixing to the tissue, stretching or straining once placed on tissue. Then creating the lesions in the tissue. The dressing can then be released to compress the lesion. The dressing can also be removed so that the natural elasticity of the skin or tissue helps appose the lesion openings. Accordingly, the dressing can be removed.

In additional variations, the dressings or frames described herein can be affixed to external structures placed on a patient to provide the degree of lift or tissue movement required for an acceptable clinical effect. For example, a cap or similar structure can be placed on a patient's head and serve an anchoring type device that allows the dressings or frames to displace tissue for an acceptable simulated visual result.

The devices, methods and kits described herein can be used for applying force to any portion of tissue to compress, reposition, or lift tissue as required by the intended cosmetic application (e.g., lifting of the breast, stretching scalp tissue to increase a density of implanted, underarm, abdominal procedures, etc. The dressing described herein can be fabricated from any biocompatible material that can provide the compressive force necessary to achieve the intended result. For example, the dressings can comprise a polymer, a shape memory polymer (e.g., acrylate-based, styrene-based and epoxy-based shape memory polymers), or biocompatible polymer (e.g., silicone). The dressings and/or frame can be transparent or opaque or have other features as required by the intended application. The strain rates of the dressings and frames described herein can range from 1% to 100% either uni-directional, uni-axial, or bi-axial. In some variations, the predetermined strain may be 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 90%, 95%, or 100%.

The fixation means described above can include any conventionally known means to secure similar dressings or frames to tissue. For example, the devices can be secured to tissue in a variety of ways (either temporarily affixed or affixed until the fixation means is removed). For example, the devices can be removably secured to the tissue with an adhesive, with a skin piercing device, or the like. Suitable adhesives include pressure sensitive adhesives, such as polyacrylate-based, polyisobutylene-based, temperature activated adhesives, chemically activated adhesives and silicone-based pressure sensitive adhesives. Suitable skin-piercing devices include clamps, needles, microneedles, sutures, anchors, staples, microtines and the like.

The devices may have any suitable or desirable shape or size. In some examples, the shape of the dressings or frames can be adjusted before, during or after the procedure. For example, the devices may have a shape selected from the group consisting of rectangles, circles, squares, trapezoids, toroids, ovals, or segments and combinations thereof. For example, some devices may be substantially circular, others may be substantially toroidal, and still others may be substantially rectangular.

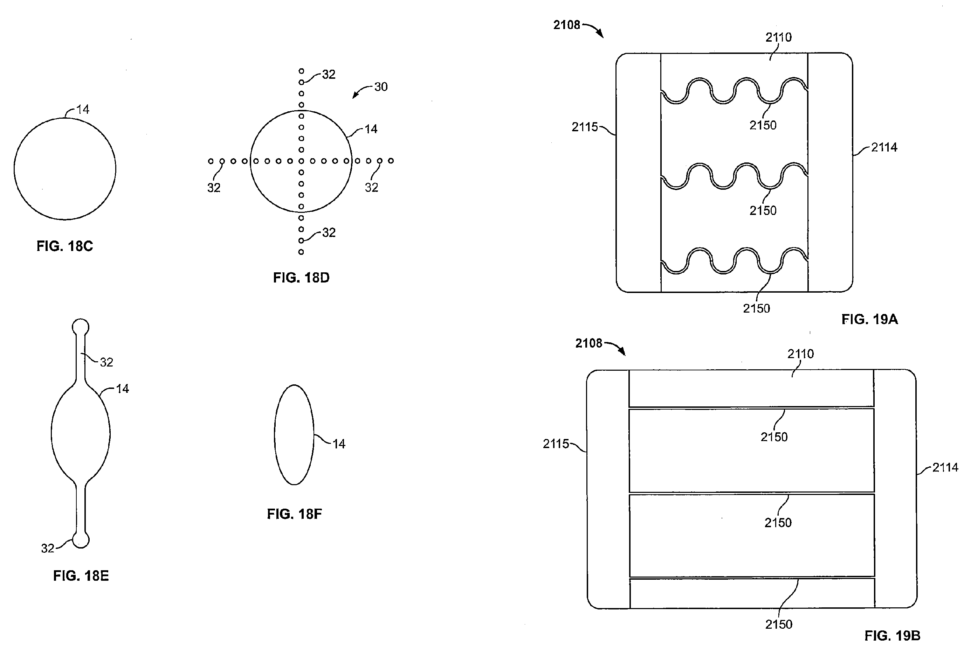

In another aspect, altering the geometry of a lesion to assist in wound healing can be combined with methods and devices described herein to improve the outcome of a treatment. For example, Fig. 17A provides a representative illustration of a top view of a lesion **14** without application of any compressive force. Fig. 17B illustrates the lesion **14** of Fig. 17A, where a compressive force, as described above and illustrated by arrows **26,** attempts to close the lesion **14.** However, the lesions **14** circular geometry creates regions of increased stress **28.** The high stress regions **28** are not desirable for a number of reasons. For example, the high stress regions **28** can impede the healing process. Moreover, the high stress regions **28** caused increased resistance when attempting to close the lesion **14.** For example, the high stress regions **28** can provide an opposing force to the dressing and ultimately render the procedure less effective. Fig. 18A shows a variation of a shaped lesion **30** intended to reduce areas of high stress when the lesion **30** is compressed as discussed herein. The lesion **30** can be created via a single treatment or can comprise a number of treatments to produce a desired shape. For example, in Fig. 18A the lesion **30** can be created to be asymmetrical so that portions **32** of the lesion **30** do not generate high areas of stress when the lesion is compressed (see e.g., Fig. 18B). In one example, a shaped lesion **30** is created via a slit in tissue and superimposing a circular lesion on the slit portion. Alternatively, as shown in Fig. 18C, a standard lesion **14** is created and then a secondary process creates one or more additional lesions **32** to create a shaped lesion **30** as shown in Fig. 18D. The creation of the base lesion **14** can be made by any conventional means(e.g. a CO2 laser) that creates a lesion of the desired size. The additional lesions **32** can be created using a means that allows creation of smaller lesions.

Fig. 18E illustrates another variation of a lesion **14** with features **32** to prevent high stress areas upon compression of the tissue. Such complex lesions can be created using a masking approach or creating series of therapeutic treatment lesions similar to that shown in Fig. 18D. Fig. 18F illustrates another lesion geometry conducive to avoiding high stress areas upon compression of tissue. In this variation, the lesion **14** includes a relatively simple geometric configuration (oval as opposed to circular).

As noted previously, the dressing may comprise an elastic member, such as a sheet of elastic material. The elastic material of the dressing may comprise a single layer of material or multiple layers of the same or different materials. The material may have any of a variety of configurations, including a solid, foam, lattice, or woven configuration. The elastic material may be a biocompatible polymer, e.g., silicone, polyurethane, TPE (thermoplastic elastomers), synthetic rubber or co-polyester material. The thickness of polymer sheets may be selected to provide the dressings with sufficient load carrying capacity to achieve desired recoverable strains, and to prevent undesired amounts of creep deformation of the dressings over time. In some variations, the thickness across dressings is not uniform, e.g., the thickness across the dressing may be varied to change the stiffness, the load carrying capacity, or recovery strains in selected orientations and/or locations. The elastic material of the exemplary dressing may have a thickness in the range of about 50 microns to 1 mm or more, about 100 microns to about 500 microns, about 120 microns to about 300 microns, or in some variations about 200 microns to about 260 microns. The exemplary dressings have an edge thickness of about 500 microns or less, 400 microns or less, or about 300 microns or less may exhibit less risk of skin separation from inadvertent lifting when inadvertently brushed against clothing or objects. In some variations, the dressings are tapered near the edges to reduce thickness. A tapered edge may also ameliorate peak tensile forces acting on skin tissue adjacent to the adhesive edges of the dressing. This may or may not reduce the risk of skin blistering or other tension-related skin trauma. In other variations, the edges of the dressing may be thicker than the middle of the dressing. It is hypothesized that in some configurations, a thicker dressing edge may provide a relative inward shift of the location of the peak tensile forces acting near the dressing edge, compared to dressings of uniform thickness. The elastic material may have a load per width of at least 0.35 Newtons per mm at an engineering strain of 60% or a load per width of at least 0.25 Newtons per mm at an engineering strain of 45%. The elastic material may have a load per width of no greater than about 2 Newtons per mm at the engineering strain of about 45% to 60%, about 1 Newtons per mm at the engineering strain of about 45% to 60%, about 0.7 Newtons per mm at the engineering strain of about 45% to 60%, or no greater than about 0.5 Newtons per mm at the engineering strain of about 45% to 60%. The system elastic material may have a load per width that does not decrease from an engineering strain of 0% to 60%, a load per width plot that increases linearly from an engineering strain of 0% to 60%, or a load per width plot that is not convex from an engineering strain of 0% to 60%. The elastic material may comprise an adhesive configured to maintain a substantially constant stress in the range of 200 kPa to about 500 kPa for at least 8 hours when strained to an engineering strain of about 20% to 30% and attached to a surface. The elastic material may comprise an adhesive configured to maintain a substantially constant stress in the range of 200 kPa to about 400 kPa for at least 8 hours when strained to an engineering strain of about 20% to 30% and attached to a surface. The substantially constant stress may vary by less than 10% over at least 8 hours, or by less than 5% over at least 8 hours.

Although the depicted dressings may have a generally rectangular configuration with a length and/or width of about 160 mm to about 60 mm, in other variations the dressing may have any of a variety of lengths and widths, and may comprise any of a variety of other shapes. Also, the corners of the dressing may be squared or rounded, for example. The lengths and/or widths of an exemplary dressing may be in the range of about 5 mm to about 1 meter or more, in some variations about 20 mm to about 500 mm, and in other variations about 30 mm to about 50 mm, and in still other variations about 50 mm to about 100 mm. In some variations, the ratio of the maximum dimension of the dressing (e.g. its length) to an orthogonal dimension to the maximum dimension (e.g. width), excluding the minimum dimension of the dressing (e.g. the thickness), may be in the range of about 1:3, about 1:2, about 1:1, about 2:1, about 3:1, about 4:1 about 5:1, about 6:1, about 7:1, about 8:1, about 9:1 or about 10:1 or greater. In some variations, the strain axis of the dressing in use may be oriented with respect to the maximum dimension or to the orthogonal dimension to the maximum dimension. In some variations, the final compressive stress and strain imposed onto the skin by the elastic material may be the result of the dynamic equilibrium between the tensile stress in the skin and the elastic material of the dressing. The skin at the skin site typically comprises an inherent tension that stretches incision site, whether or not any tissue was excised from the skin site. The elastic material and the adhesive region may be configured to be applied to a skin location so that when the dressing is stretched to a particular tension and then adhered to the incision site, tensile stress in the dressing is transferred to the incision site to compress the tissue directly under the dressing along a tangential axis to the skin surface, the stress and strain imposed onto the skin location has a net or resultant orientation or axis is also generally tangential or planar to the elastic material and/or the outer surface of the skin location, with a similar axis to the orientation or axis of the tensile stress in the dressing. The tension in the dressing will relax to a tension level that maintains equilibrium with increased tension in the skin adjacent to the dressing. The application of the dressing to the skin location may involve the placement of the dressing without overlapping or being wrapped onto itself, e.g. wherein only adjacent regions of the dressing are interconnected and wherein non-adjacent regions of the dressing are not interconnected. The actual amount of stress and strain imposed on the skin may vary, depending upon the particular person, skin location, the thickness or various mechanical characteristics of the skin layers (e.g. epidermis, dermis, or underlying connective tissues), and/or the degree of pre-existing scarring, for example. In some further variations, the wound treatment dressing may be selected or configured for use at a specific body location, such as the scalp, forehead, cheek, neck, upper back, lower back, abdominal region, upper torso (including but not limited to the breast folds), shoulder, upper arm, lower arm, palm regions, the dorsum of the hand, finger, thigh, lower leg, the dorsum or plantar surface of the foot, and/or toe. Where applicable, some body regions may be further delineated into anterior, posterior, medial, lateral, proximal and/or distal regions, e.g. the arms and legs.

The dressing may be configured to impose a skin strain in the range of about 10% to about 60% or more, in other configurations about 15% to about 50%, and in still other configurations, about 20% to about 30% or about 40%; the dressing may also be configured to impose a strain of less than 10%. To achieve the desired degree of skin strain, the dressing may be configured to undergo elastic tensile strain in the range of about 20% to about 80% or more, sometimes about 30% to about 60%, and other times about 40% to about 50% or about 60%. The dressing may comprise any of a variety of elastic materials, including but not limited to silicones, styrenic block copolymers, natural rubbers, fluoroelastomers, perfluoroelastomers, polyether block amides, thermoplastic elastomers, thermoplastic polyurethane, polyisoprene, polybutadiene, and the like. The material of the exemplary dressing may have a Shore A durometer in the range of about 20 to about 90, about 30 to about 80, about 50 to about 80. The exemplary dressing was constructed of MED 82-5010-05 by NUSIL TECHNOLOGY LLC (Carpinteria, CA). Other examples of suitable materials are described in U.S. Appln. No. 11/888,978.

When the dressing is applied to a skin location and allowed to at least partially recover to its base configuration, the recovery level or equilibrium level of strain in the dressing may be in the range of about 4% to about 60% or more, in other configurations about 15% to about 50%, and in still other configurations, about 20% to about 30% or about 40%. The ratio between the initial engineering tensile strain placed onto the dressing before recovery and the resulting engineering compressive strain in the skin may vary depending upon the skin type and location, but in some examples, may be about 2:1. In other examples, the ratio may be in the range of about 4:1 to about 5:4, about 3:1 to about 5:3, or about 5:2 to about 2:1. These skin strain characteristics may be determined with respect to a reference position of the body or body part, e.g. anatomical position, to facilitate reproducible measurements. The particular degree of strain may be characterized as either an engineering strain or a true strain, but may or may not be calculated based upon or converted from the other type of strain (e.g. the strain may be based upon a 45% engineering strain that is converted to a true strain).

In some further variations, one or more characteristics of the elastic material may correspond to various features on the stress/strain curve of the material. For example, the engineering and true stress/strain curves for one specific example of the dressing comprises a material that exhibits an engineering stress of about 1.2 MPa at about 60% engineering strain, but in other examples, the engineering stress may be in the range of about 900KPa to about 3.5MPa, about 1 MPa to about 2.2MPa, about 1 MPa to about 2MPa, about 1.1 MPa to about 1.8 MPa, about 1.1 MPa to about 1.5 MPa, about 1.2 MPa to about 1.4 MPa. When unloading or relieving stress from the dressing, the material may be configured with an engineering stress of about 380 KPa at about 40% engineering strain, but in other examples, the engineering stress during unloading of the material to about a 40% strain may be in the range of about 300 KPa to about 700 KPa, about 325 KPa to about 600 KPa, about 350 KPa to about 500KPa, or about 375 KPA to about 425 KPa. When unloading the material to an engineering strain of about 30%, the material exhibits an engineering stress of about 300 KPa, but in other examples, the engineering stress when unloading the material to about 30% strain may be in the range of about 250 KPa to about 500 KPa, about 275 KPa to about 450 KPa, about 300 KPa to about 400KPa, or about 325 KPA to about 375 KPa. When unloading to an engineering strain of about 20%, the material may have an engineering stress of about 100 KPa, but in other examples, the unloading engineering stress at about 20% may be in the range of about 50 KPa to about 200 KPa, about 75 KPa to about 150 KPa, or about 100 KPa to about 125KPa. In some examples, the material may be configured to at least achieve a specific range or level of engineering stress at each of the specified engineering strain levels described above, but in other examples, the material may be configured for lower levels of maximum engineering strain, e.g. up to about 30% or about 40%.

In some examples, certain portions of the stress/strain curve may have a particular morphology. For example, for a particular level of maximum strain the loading curve may be generally linear on the corresponding true stress/strain curve. In an example using a dressing described herein, up to a true strain of about 45%, the loading curve had a generally linear configuration. In other examples, the configuration may only be linear along a portion of the loading curve or may be curved along the entire loading curve. Where the loading curve is non-linear, the loading curve may be convex, concave or both. Also, in some examples, the tangent line of the loading curve (i.e. the line between the two triangles) may also be generally co-linear.

In some variations, the elastic material comprises a material having an elastic modulus E of at least about 1 MPa, about 1.5 MPa, about 2 MPa, about 2.5 MPa, about 3 MPa, about 3.5 MPa, about 4 MPa, about 5 MPa, about 6 MPa, about 7 MPa, about 8 MPa, about 9 MPa or at least about 10 MPa or greater. The material elastic modulus E may be no greater than about 10 MPa, about 9 MPa, about 8 MPA, about 7 MPa, about 6 MPa, or about 5 MPa, or about 4 MPa.

In addition to the absolute stress levels at certain strain levels described above, the material may also be characterized with respect to the ratio between a) the stress to achieve a particular strain during loading, and b) the stress at the same strain during unloading. For example, the material may have a ratio of at least 4:1 to about 3:2 at each of the 20%, 30% and 40% strain levels, but in other examples, the material may exhibit these ratios only at 20%, at 30%, or at 40% strain levels, or at both 20% and 30% but not 40%, or at both 30% and 40% but not 20%. In other examples, the ratio at one, some or all of the strain levels may be in the range of about 3:1 to about 2:1, or about 5:2 to about 2:1.

In some examples, the elastic material of the dressing may be configured under testing conditions to achieve a stable level of stress at a constant strain, e.g. the material exhibits a limited amount of stress relaxation over a particular period of time and at a particular level of strain. The period of time may be at least about 8 hours, about 12 hours, about 18 hours, about 24 hours, about 36 hours, about 48 hours, about 72 hours, about 4 days, about 5 days, about 6 days, or about a week or more. The level of strain may be about 10%, about 20%, about 30%, about 40%, about 50%, about 60%, about 70%, or about 80% or more. The stress of the exemplary dressing over various time curves may be configured to maintain an engineering stress of about 300 KPa at an engineering strain of about 30% without noticeable deviation over a period of about 1 hour, about 2 hours, about 3 hours, about 4 hours, about 5 hours, about 6 hours, about 7 hours, or about 8 hours or more. The stresses at 10% strain, 20% strain, and at 40% may be lower or higher.

In some variations, the elastic material or the dressing may be configured under testing conditions to maintain a particular minimum level of stress when held at a constant strain over a particular time period. In an example to assess the ability of a backing material to maintain a stress and strain on skin over time, engineering strains were measured while each backing material was tensile strained to 60% at a rate of 100 microns per second and held for 10 minutes, and then dropped to a strain of 30% at a rate of 100 microns per second and held for 9 hours. For example, the exemplary dressing is able to maintain an engineering stress level of about 350 KPa at an engineering strain of 30%. In some other examples, the minimum level of stress may be about 100 KPa, about 120 KPa, about 140 KPa, about 160 KPa, about 180 KPa, about 200 KPa, about 220 KPa, about 240 KPa, about 260 KPa, about 280 KPa, about 300 KPa, about 320 KPa, about 340 KPa, about 360 KPa, about 380 KPa, about 400 KPa, about 420 KPa, about 440 KPa, about 460 KPa, about 480 KPa, about 500 KPa, about 600 KPa, about 700 KPa, about 800 KPa, about 900 KPa or about 1000 KPa or greater. The level of constant strain may be different in other configuration, with a level of about 15%, about 20%, about 25%, about 30%, about 35%, about 40%, about 45%, about 50%, about 55%, about 60%, about 65%, about 70%, about 75%, or about 80%. The time period over which the dressing is able to maintain a stress level may be at least about 2000 seconds, about 3000 seconds, about 4000 seconds, about 5000 seconds, about 6000 seconds, about 7000 seconds, about 8000 seconds, about 9000 seconds, about 10000 seconds, about 20000 seconds, about 30000 seconds, about 40000 seconds, about 50000 seconds, about 60000 seconds, about 70000 seconds, about 24 hours, about 36 hours, about 48 hours, about 72 hours, about 4 days, about 5 days, about 6 days, about 7 days, about 10 days, about 2 weeks, about 1 month or more. In some variations, the dressing, the elastic material and/or the adhesive material is configured to exhibit less than about a 15% change in stress or strain level over the particular period when applied to a skin surface or test surface. In other examples, the degree of change may be about 12%, about 10%, about 8%, about 6%, about 5%, about 4%, about 3%, or about 2% or less. The stress or strain may be an engineering stress or strain, and/or a true stress or strain.

The adhesive used may be, for example, a pressure activated adhesive (PSA), as a silicone, acrylic, styrene block copolymer, vinyl ether, nitrile or other PSA. In other variations, a non-pressure sensitive adhesive may be used, including but not limited a heat or light-cured adhesive. The pressure sensitive adhesive may be made from, e.g., polyacrylate-based, polyisobutylene-based, silicone-based pressure sensitive adhesives, synthetic rubber, acrylic, and polyisobutylene (PIB), hydrocolloid, and the like. The T-peel release force and blunt probe tack force of the adhesive may be measured by a standardized test method, such as ASTM D1876 and ASTMD2979 or other appropriate method. In some variations, the T-peel release force or blunt probe tack test value of the adhesive is configured to maintain loads of at least about 50 mPa/mm for at least about 24 hours, about 48 hours, about 72 hours, about 1 week, about 2 weeks, about 3 weeks, about 4 weeks or more. In other variations, the loads may be at least about 75 mPa/mm, about 100 mPa/mm, about 125 mPa/mm, or at least about 150 mPa/mm over the particular time period. The degree of adhesion (e.g. as measured by the T-peel release force or blunt probe tack test value) may vary depending upon the degree of strain placed onto the skin or incision site, and in some variations, these time periods may be based upon an average skin strain of about 10%, about 20%, about 30%, about 40%, or about 50% or more. In some variations, the adhesive may have a T-peel release force of at least about 150 kg/m, about 160 kg/m , about 170 kg/m , about 180 kg/m, about 190 kg/m, about 200 kg/m, about 210 kg/m, about 220 kg/m, about 230 kg/m, about 240 kg/m, about 250 kg/m, about 260 kg/m, about 270 kg/m, about 280 kg/m, about 290 kg/m, about 300 kg/m, about 310 kg/m, about 320 kg/m, about 330 kg/m, about 340 kg/m, about 350 kg/m, about 400 kg/m, about 450 kg/m, or at least about 500 kg/m or higher. In some further variations, the T-peel release force may be no greater than about 1000 kg/m, about 900 kg/m, about 800 kg/m, about 700 kg/m, about 600 kg/m, about 500 kg/m, about 400 kg/m or about 300 kg/m. The blunt probe tack test value of the adhesive may be at least about 0.50 kg, about 0.55 kg, about 0.60 kg, about 0.65 kg, about 0.70 kg or about 0.75 kg or higher, and may be no greater than about 1 kg, about 0.9 kg, about 0.8 kg, about 0.7 kg, or about 0.6 kg. The T-peel release force and blunt probe tack force may be measured by a standardized test method, such as ASTM D1876 and ASTMD2979 or other appropriate method. Other features or variations of the device are described in U.S. Appl. No. 11/888,978, filed on August 3,2007.

Release liners may be provided over the skin adhesive and may be removed prior to stressing , straining , stretching and/or applying a dressing to a subject. The release liners may comprise any of a variety of materials, including both opaque and transparent materials. The release liners may comprise Mylar or paper, or any other material with reduced adhesion to the adhesive material(s) of the device. For example, for a silicone adhesive, a fluoropolymer-treated polyester film may be used, and for an acrylic pressure sensitive adhesive, a silicone treated polyester or Mylar film or silicone treated craft paper may be used. In variations where the device has multiple separate adhesive regions, separate release liners may be provided for each region, or some regions may be covered by the same release liner.

In some variations the assembly may comprise one or more mechanisms or elements configured to facilitate separation, release, removal or detachment of the dressing from the packaging, manipulation elements applicator or tensioning device, other attachment elements or other portions of the dressing assembly, or other elements of the devices. Release elements or releasable attachment structures may include but are not limited to pockets and tabs, hook and loop mechanism, hooks, angled bars, pivoting, rolling, rocking or sliding features associated with or coupled to attachment structures , adhesives, removable adhesives, adhesive tapes or other adhesive devices, pegs, rip cords, towel bar configurations, sliding pins, friction locks, cam locks, vacuum or suction devices, snap connectors, carpet tack, press fit connections or other connections, levers, latches, locking members, spring members, for example, or other mechanisms such as cutters or rip cords or other structures or features to facilitate tearing, cutting or separation of attachment structures or elements perforated or otherwise severable structures, that permit removal of dressing from the applicator ,packaging, other portions of the dressing assembly and/or attachment structures, features, elements or portions. They may be self-releasing latches or spring members. They may be actuated when a pressure member is applied to a skin treatment device prior to removing the applicator. They may be manually actuated.

Packaging devices, applicators, tensioning devices, and corresponding attachment features may be configured to provide multi-direction strain or additional strain in an orthogonal direction to a dressing.

The packaging device, manipulation elements, applicator, tensioning device and/or attachment structure profile may be straight, curved or otherwise varied. For example, the shape of the elements of a device may be configured to follow the shape of the area of the subject's body to which the skin treatment device is to be attached. A packaging device, manipulation elements, tensioning device, applicator or other elements thereof may be selected or configured to have a profile that has a desirable profile for a particular body location or profile where the skin treatment device is to be placed on a subject's skin. A packaging device, manipulation element, applicator, tensioning device or elements thereof may be selected or configured to closely match a portion of a subject's body profile. The packaging device, manipulation element, applicator or tensioning device and/or an element or segment thereof, may be curved, curvable, flexible, bendable, malleable, deformable, shapeable or movable to provide alternative shapes or profiles of an attached dressing. They may be relatively curved, curvable, flexible, malleable, bendable, deformable, shapeable or movable in at least one direction while being more rigid in another direction.

A variety of locking, latching, securing, attaching or detent mechanisms may be used to maintain the dressing, packaging, manipulation elements, applicator and/or tensioning device in a various configurations including but not limited to unstrained, partially strained, strained configurations. A variety of locking, latching or detent mechanisms may be used to maintain a dressing in a variety of configurations including unstrained, partially strained, strained. By locking the packaging, applicator, tensioning device manipulation elements or other elements coupled to the dressing, or dressing in a strained position, a predetermined strain of a given dressing may be achieved and maintained until released. The predetermined amount of strain may be a predetermined absolute percentage of strain or level of force that is independent of the shape and/or size of the treatment site.

According to a variation, a skin treatment device is provided that may be strained prior to application to the skin of a subject. According to variations, device or device elements may provide a variable strain to the skin treatment device.

According to variations, a skin treatment device may comprise one or more manipulation elements removably coupled to a dressing. Such variations may further comprise a strain limiter that limits or determined the amount of strain applied to a dressing. Such variations may also further comprise a strain indicator that indicates a desired strain level has been reached.

According to a variation, a plurality of strain indicators may be provided where each indicator indicates a different strain level or amount.

According to a variation, a skin treatment device is provided that may be strained by a user prior to application to the skin of a subject. According to a variation, skin treatment device may include strain indicator that indicates when the dressing has been stretched by the user to a desired degree.

According to a variation, a skin treatment device that may be strained by a user may include a strain limiter configured to prevent over straining of the device, or straining the device beyond a desired degree or amount.

According to a variation, the strain limiting elements may prevent straining in regions where straining or less strain is desired. The strain limited skin treatment devices may also be shaped to provide a graduated strain or varying strain through the strained device. The device may be stretched by hand or may otherwise be configured to be stretched with a tensioning device, for example a set forth in Application Serial Nos. 12/854,859 and 13/345,524.

According to a variation, a skin treatment device comprises one or more relaxed threads, strings, wires or other elongate, elongatable, straightenable or stretchable members that straighten, lengthen and/or stretch to a desired amount, degree, and/or preset limit. For example, in one variation, the members may have an undulating shape when the skin treatment device is unstretched and a straight configuration when the skin treatment device is stretched to a desired degree. The members may have a sufficient tensile strength to prevent over-stretching of the skin treatment device. According to a variation the skin treatment device is constructed of multiple layers of an elastic material such as silicone with an adhesive between layers to which the members are attached initially in the relaxed, undulating, sinusoidal, unstraightened or other unstretched configuration. According to a variation, the shape limiting strings or other devices may prevent straining in regions where straining or less strain is desired. For example, the threads may be straight at the edges of the skin treatment device to prevent straining at the edges.

Figs. 19A and 19B illustrate a strainable skin treatment device 2110 with strain indicators and/or strain limiters 2150. The strain indicators and/or limiters 2150 may be visibly positioned in or on a device assembly 2108 which may comprise layers of elastic material sandwiching the strain indicators 2150 therebetween. The material may or may not be transparent or translucent. Indicators may also be positioned on the surface of the device. The strain indicators and/or limiters 2150 may be threads, wires or other elongate or elongatable members 2155. In use, the device assembly 2108 is grabbed by a user at opposing sides 2114, 2115 and stretched. The opposing sides 2114, 2115 may also have removable (or non-removable) manipulation elements attached to the ends to provide for a more even or uniform strain, and may comprise an inelastic material which is the same or different as the strain indicators/limiters 2150. The manipulation elements may span the entire transverse dimension of the device 2110 to the axis of tensioning, as depicted in Fig. 19B, but in other variations may be less than the entire transverse dimension (e.g. having a transverse dimension sufficient to span a plurality of strain indicators/limiters 2150, but less than the full transverse dimension of the device 2110). Such elements may comprise planar members, handle members, flexible members and/or inflexible members. They may be attached and removed in a variety of manners, for example as described herein. Also, although the example depicted in Figs. 19A and 19B depict a plurality of strain indicators/limiters 2150 that are equally spaced apart and have uniform lengths and uniform attachment points across a transverse dimension to the tensioning axis of the device 2110, in other examples, the indicators/limiters 2150 may have a variable or non-uniform spacing, may have non-uniform lengths, non-uniform attachment points, and may also be serially arranged along the tensioning axis.

As the skin treatment device 2110 is stretched or strained, the device width increases and the members 2155 straighten. When the skin treatment device 2110 has been stretched or strained to a desired amount or a pre-determined or preset amount, the indicators and/or limiters 2150 are in a visible or identifiably straightened configuration, for example as shown in Fig. 19B. The indicators and/or limiters 2150 may also comprise a relatively inelastic material such as , e.g. a nylon string, that prevents or resist the skin treatment device 2110 from over stretching or straining to a degree greater than is desirable, thus limiting the strain applied to the device 2110. A skin adhesive with a protective liner may be applied to a side of the skin treatment device 2110 prior to stretching, stressing or straining. The liner may be removed from the skin treatment device 2110 prior to stretching. According to one aspect of the invention, it is believed that a range of strain is significant in providing treatment to a skin. According to variations other shapes or stretching indicators may be used where a visible change in an indicator line, shape or color may occur. Examples of a color change material or structure are described in U.S. Pub. No. 2006/0246802 to Hughes et al.

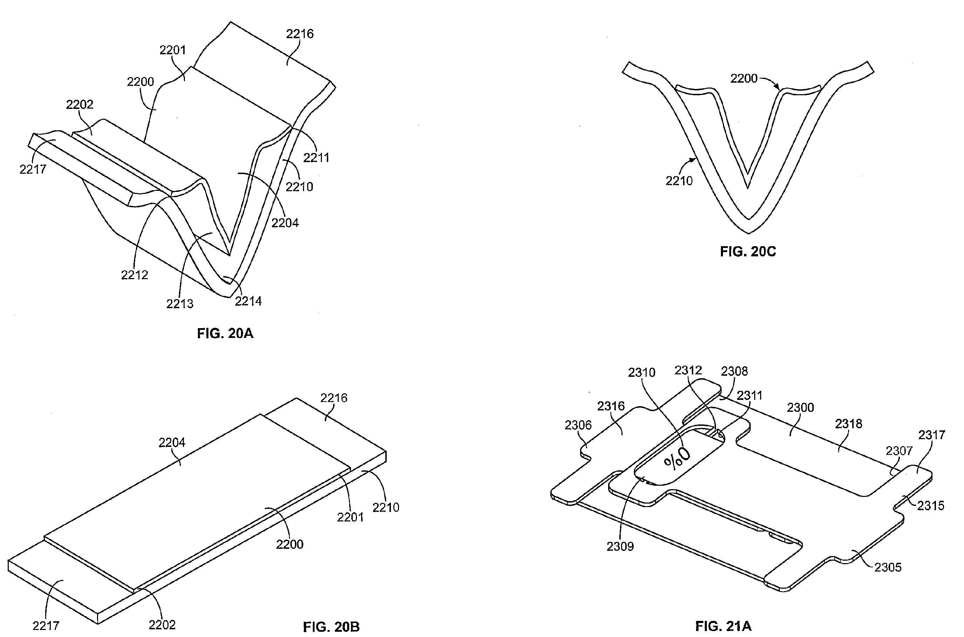

Figs. 20A to 20C illustrate a manually strainable skin treatment device 2200 that may comprise, an elastic material. The skin treatment device 2200 is coupled to a strain limiting backing 2210 comprising a flexible inelastic or relatively less elastic material with respect to the elastic material of the skin treatment device 2200, such as, for example, LDPE, FEP or nylon. As shown in Fig. 20A, prior to straining, the skin treatment device 2200 is shown in a relaxed, folded configuration, coupled (e.g. with an adhesive such as a preferentially removable adhesive as compared to skin adhesive used on surface 2204) at its opposing ends 2201, 2202 to the inner surface 2214 of the strain limiting backing 2210 at locations or areas 2211, 2212. In the relaxed configuration the length of the device 2200 is a first shorter length. The ends 2216, 2217 of the strain limiting backing 2210 may extend outward of locations 2211,2212 and be used as grips to pull and straighten the backing 2210 as shown in Fig. 20A to thereby strain and increase the length of the device 2200 to a second greater length. The inner surface 2214 of the strain limiting backing may comprise an adhesive layer such as a preferentially removable adhesive as compared to skin adhesive used on surface 2204 which may attach the back 2203 of the device 2200 to the inside surface 2214 of the backing 2210 when the device is strained. The exposed surface 2204 of the device 2200 may comprise a skin adhesive such as a pressure sensitive adhesive that secures the device to a skin surface when applied. After the device is applied to a skin surface, the backing 2210 may be peeled away from the device 2200.

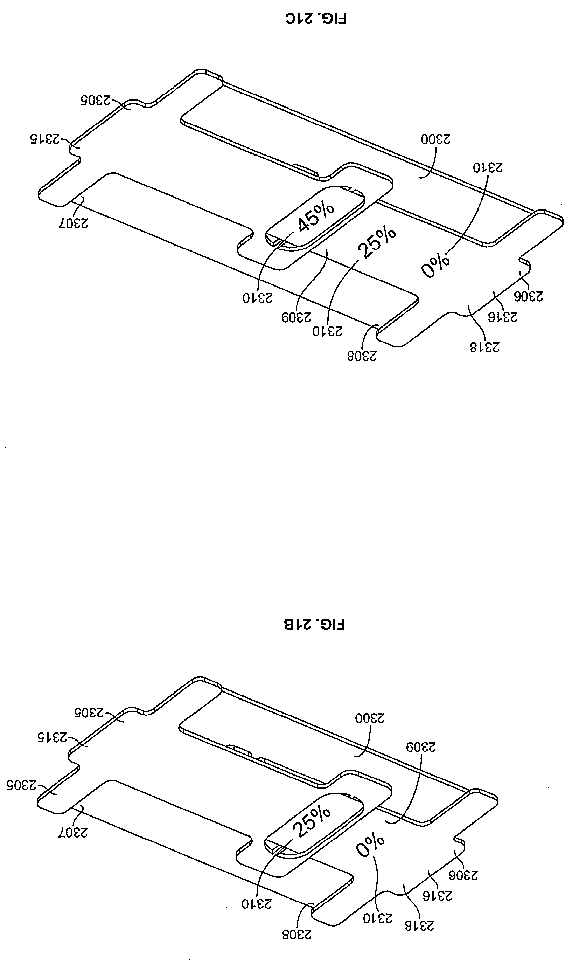

Figs. 21A to 21C illustrate a strainable skin treatment device 2300 which may be strained and/or stretched by exerting a tensioning force on the ends 2307, 2308 of the skin treatment device 2300. A first opposing sliding element 2305 is coupled at its end 2317 to the first end 2307 of the device 2300 and a second opposing sliding element 2306 is coupled at its end 2316 to the second end 2308. The sliding elements 2305 may be removable coupled to the skin device 2300, for example with an adhesive such as a preferentially removable adhesive as compared to skin adhesive used on skin interfacing surface. The first sliding element 2305 and second sliding element 2306 are slidably coupled to each other. The first sliding element 2305 comprises a window 2311 and securing bar 2312. The second sliding element 2306 comprises an elongate tab 2309 having strain value indicators 2310 on the top surface of the elongated tab 2309 so that they may be viewed through window 2311. The tab 2309 of the second sliding element 2306 is positioned under the first sliding element 2305 and through the window 2311 of the first sliding element 2305. The securing bar 2312 is positioned across the window 2311 and below the tab 2309, to hold the tab 2309 in position through the window 2311. Additional securing elements such as rails or guide elements may or may not be used to guide or maintain connection or alignment of the tab 2309 with respect to the window 2311. The ends 2306, 2307 and/or ends 2316, 2317 may be used to grip and strain the device 2300, and may or may not comprise an inelastic material. As the skin treatment device 2300 is strained, the elements 2305, 2306 slide apart and the tab 2309 slides through the window 2311. In the first position in Fig. 21A, the device is at a 0% strain in a first unstrained configuration. In Fig. 21B, the device 2300 is strained about 25% as indicated by the strain indicator 2310. In Fig. 21C the device is strained to 45% as indication by the indicator 2310. A user may select the amount of strain desired. A user may use the indicators 2310 to strain the device 2300 to a desired degree. A skin adhesive may be provided on the skin attachment side 2314 of the device 2300. The ends 2316, 2317 may be attached to the top surface 2318 (opposing a skin adhesive surface) of the device 2300 by way of an adhesive, such as a preferentially removable adhesive as compared to skin adhesive used on the skin adhesive surface After straining the device 2300 a desired amount, it may be placed on a skin surface and the manipulation elements or sliding members 2316, 2317 may be removed. Alternatively, one of the sides 2307 or 2308 of the device may be applied to a skin surface. The device 2300 may be strained a desired degree by pulling on the on the unattached one of the sides 2307 or 2308 while observing the indicia 2310. Once a desired strain level is reached the unattached side 2307 or 2308 may be attached to the skin and the manipulation elements or sliding members 2316, 2317 may be removed

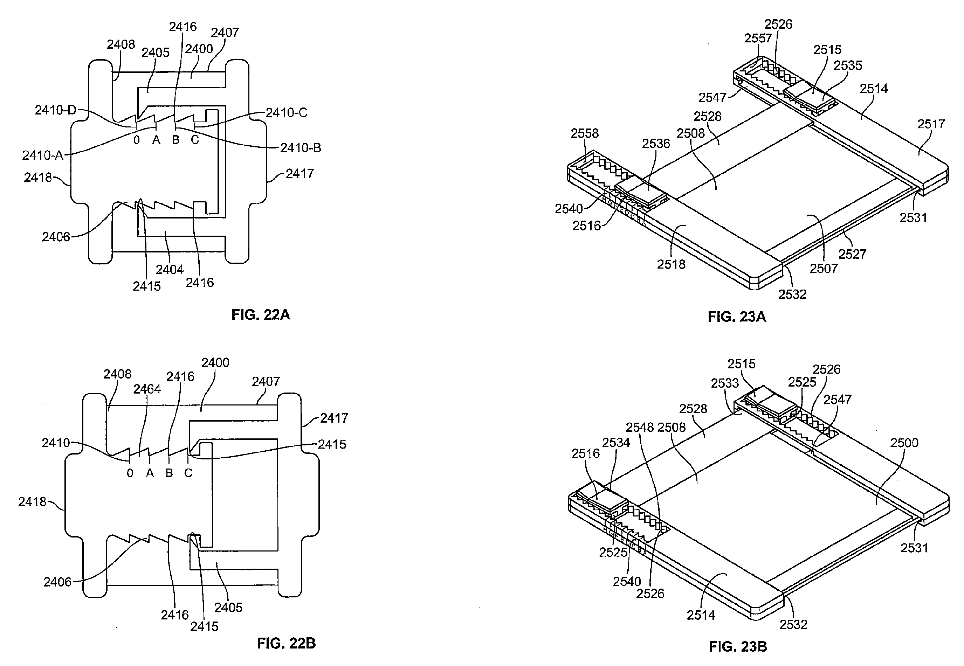

Figs. 22A and 22B illustrate a strainable skin treatment device 2400 which may be strained and/or stressed by exerting a tensioning force on the ends 2407, 2408 of the skin treatment device 2400. A ratchet 2404 comprises first opposing ratchet element 2405 coupled at its end 2417 to the first end 2407 of the treatment device 2400, and a second opposing ratchet element 2406 coupled at its end 2418 to the second end 2408 of the treatment device 2400. Ratchet elements 2405, 2406 are movably coupled to each other so that they may be separated and the device may be tensile stressed and maintained in a strained configuration. The ends 2407, 2408 of the skin treatment device and/or ends 2417, 2418 of the ratchet elements 2405, 2406 may be used to grip and strain stress and/or stretch the device 2400. The first ratchet element 2405 comprises pawls 2415 that engage sloped teeth 2416 of the second ratchet element 2406 and prevent movement backwards, loss of strain and/or complete relaxation of a tensile stressed skin treatment device 2400. As the ends 2417, 418 of the ratchet 2404 move apart, the skin treatment device 2400 is strained. Strain indicators or indicia 2410 are on the second ratchet element 2406 adjacent the teeth 2416 and indicate a degree of strain of the device 2400 based on which one of the teeth 2416 engages the pawl 2415. In the first position in Fig. 22A, the device 2400 is relatively unstrained and in the position indicated by strain indicator "0" 2410-0. In Fig. 22B, the device is strained to a maximum amount as indicated by the strain indicator "C" 2410c. Positions 0, A, B, and C 2410-0, 2410a, 2410b and 2410c correspond to different teeth or positions on the ratchet 404 and accordingly, different strain amounts or zero strain. A user may select the amount of strain. A user may use the indicators 2410 to strain the device 2400 to a selected or desired amount. A skin adhesive may be provided on the skin attachment side of the device 400 to adhere the device 2400 to the skin. The ends 2417, 2418 may be attached to the top surface 2419 of the device 2400 by way of an adhesive, such as a preferentially removable adhesive as compared to skin adhesive used on skin interfacing surface. The ratchet elements 2405, 2406 may be peelable from the device or otherwise removably coupled to the device 2400. After the device is applied to a subject, the ratchet elements 2405, 2406 may be removed leaving the device 2400 on the skin. In other variations, one or more of the ratchet elements and/or pawl elements may be left in place or otherwise configured to be non-removable. In some variations comprising removable elements used only during the application of the device, the removable elements may be attached to the device using adhesives comprising a higher shear force resistance but a lower T-peel force resistance, relative to the same force.

Figs. 23A and 23B illustrate a strainable skin treatment device 2500 which may be strained , stretched and/or tensile stressed by exerting a tensioning force on ratcheted tabs 2515, 2516 or other manipulation elements of a straining structure 2514. The skin treatment device 2500 is coupled at its first end 2507 to a first attachment element 2527. The first attachment element 2527 is coupled at its sides 2531, 2532 to side supports 2517, 2518 of the straining structure 2514. The second and opposite end 2508 of the skin treatment device 2500 is coupled to a second attachment element 2528. The sides 2533, 2534 of second attachment element are slidably positioned in slots 2547, 2548 formed in side supports 2517, 2518 along a portion of the length. Ratcheted openings 2557, 2558 are formed through the top surface 2550 of side supports 2517, 2518 along the portion of the length of the slots 2547, 2548. The tabs 2515, 2516 are coupled through openings 2557, 2558 to sides 2533, 2534 respectively of attachment element 2528 within the slots 2547, 2548 respectively The ratchet 2504 comprises the teeth 2526 of the ratcheted openings 2547, 2548 and pawls 2525 formed in sides of the tabs 2515, 2516. Pawls 2525 may be disengaged from the teeth 2516 by depressing the buttons 2535, 2536 on the tabs 2515, 2516 when straining the device 2500. The pawls 2525 engage the teeth 2526 when the buttons 2535, 2536 are released to maintain the device 2500 in a strained configuration by pushing buttons 2535, 2536 to release pawls 2525. The buttons 2535, 2536 may be biased by a spring in a pawl engaging direction. When buttons 2535, 2536 are released at a selected position, a desired device strain is maintained or locked in.

The tabs 2515, 2516 may be extended to strain the attached dressing. Strain indicators or indicia 2540, for example as described herein may be provided on the straining structure 2514 or device 2500 to indicate a degree of strain of the device 2500. The device may be strained a selectable amount based on which ones of the teeth 2516 engage the pawls 2515. A user may use the indicators 2540 to strain the device 2500 to a selected or desired amount. A skin adhesive may be provided on the skin attachment side of the device 2500 to adhere the device 2500 to a subject. The attachment elements 2527 and 2528 may be attached to the treatment device 2500 by a preferentially removable adhesive as compared to skin adhesive used on skin interfacing surface of the device 2500. The tabs 2515, 2516 are coupled to the attachment element 2528. The straining structure may be removable from the device 500 leaving the device 2500 on the skin.

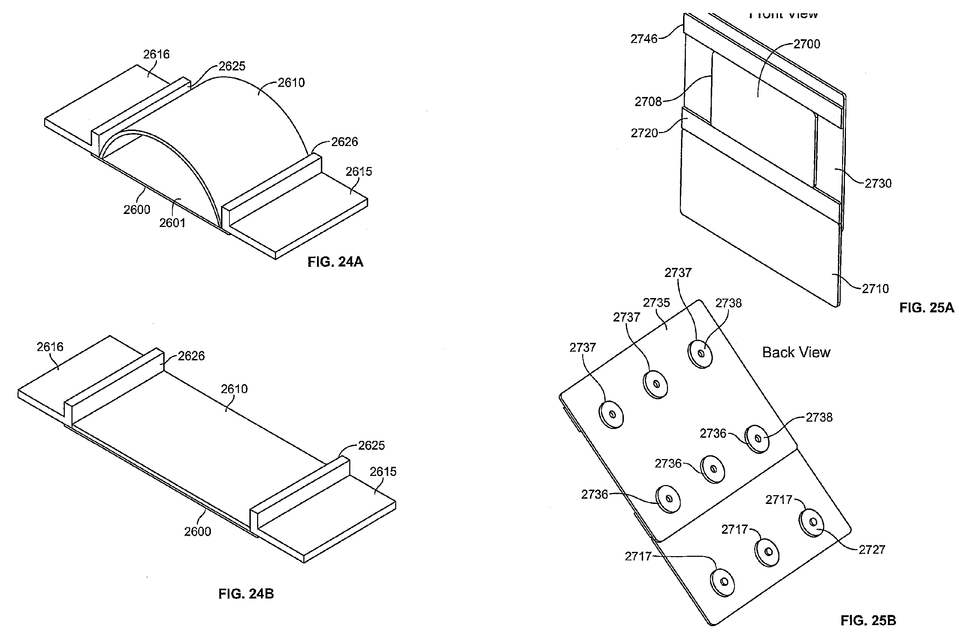

Figs. 24A and 24B illustrate a strainable skin treatment device 2600 with handles 2615, 2616 coupled to a first surface 2601 of a treatment device 2600, for example, with a peelable adhesive. The skin treatment device 2600 may be strained and/or tensile stressed by exerting a tensioning force on handles 2615, 2616. A strain limiter 2610 is coupled to inner edges or walls 2625, 2626 of the handles 2615, 2616, respectively, for example by adhering, welding, or overmolding. The strain limiter 2610 comprises at least one element that is bent, folded, arced or otherwise shaped in a first configuration, for example, as shown for example, in Fig. 24A where the distance between the inner walls 2625,2626 is less than when the device 2600 is strained. When a user manually strains the device by separating the handles 2615, 2616, the strain limiter, straightens and maintains the strain by engaging the side walls 2625, 2626 in a straight configuration. The strain limiter 2610 may be constructed for example, of LDPE, FEP, nylon, metal. The strain limiter 2610 may be coupled to the side walls of the handles 2615, 2616. The strain limiter 2610 and handles 2615, 2616 may be released from the device 2600 after it is applied to a skin surface by peeling it away.

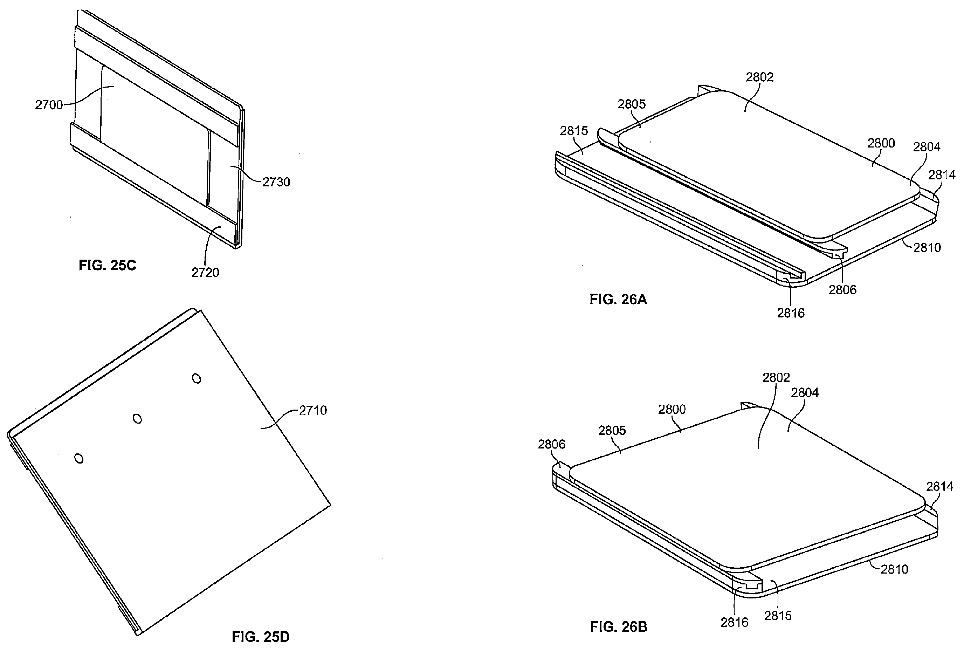

Figs. 25Ato 25D illustrate a strainable skin treatment device 2700. The treatment device 2700 is coupled to a sheet 2710 at a first side 2705 of the device, for example by way of an adhesive or other securing element 2720 capable of resisting shear stresses during straining, e.g., KAPTON® polyimide tape (DuPont, Wilmington, DE). The device 2700 may be anchored at a second end 2708 to a support structure or backing 2730 on a first surface 2733 of the backing 2730, for example by way of a securing element 2740 such as a KAPTON® tape or adhesive that is removable from the device 2700. The device 2700 may be manually or otherwise strained by grasping or pulling the sheet 710 in a straining direction. A second opposing surface 2735 of the backing 2730 comprises a first row 2736 of snap buttons 2738 and a second (or more) row 2737 of snap buttons 2738 that engage with a row 2717 of mating snap buttons 2727 on the sheet 2710. After the device 2700 is strained by tensioning or pulling the sheet 2710. The sheet 2710 may be folded back so that the row 2717 is aligned with a row of snap buttons on the backing 2730. Each row may be positioned so that a known amount of strain is created in the dressing depending on which row is used. For example, the first row 2736 of buttons 2738 would be commensurate with an amount of strain that is less that the amount of strain commensurate with the second row 2737 of buttons 2738. Accordingly a user may select an amount of strain.

Figs. 26A and 2666B illustrate a strainable skin treatment device 2800. The skin treatment device 2800 is coupled on a first side 2804 to a corresponding first side 2814 of a stiffer or more rigid backing 2810. A coupling element 2806 is coupled to the second side 2805 of the treatment device 2800. The coupling element 2806 engages or snaps into a receiving element 2816 on the second side 2815 of the backing 2810. A user may manipulate the coupling element 2806 to strain the treatment device 2800 and then attach the coupling element 2806 to the receiving element 2816. The coupling element 2806 and engaging element 2816 may be snapped together and apart for example as with press fit a tongue in groove type connector arrangement. They also may be slid apart in a planar direction with respect to the treatment device 2800 and the backing 2810. The skin treatment device 2800 and the backing 2810 may be removably coupled on the first sides 2804, 2814 by way of a peelable adhesive or other coupling mechanism. For example, the first sides 2804, 2814 may be coupled in a similar manner as coupling element 2806 and receiving element 2816. Thus, after straining and attaching the treatment device 2800 to the backing 2810 in a strained configuration, and after applying an adhesive side 2802 of the strained device 2800 to skin to be treated, the backing 2810 may be released from the device 2800 by snapping or sliding the backing 2810 apart from the device 2800.

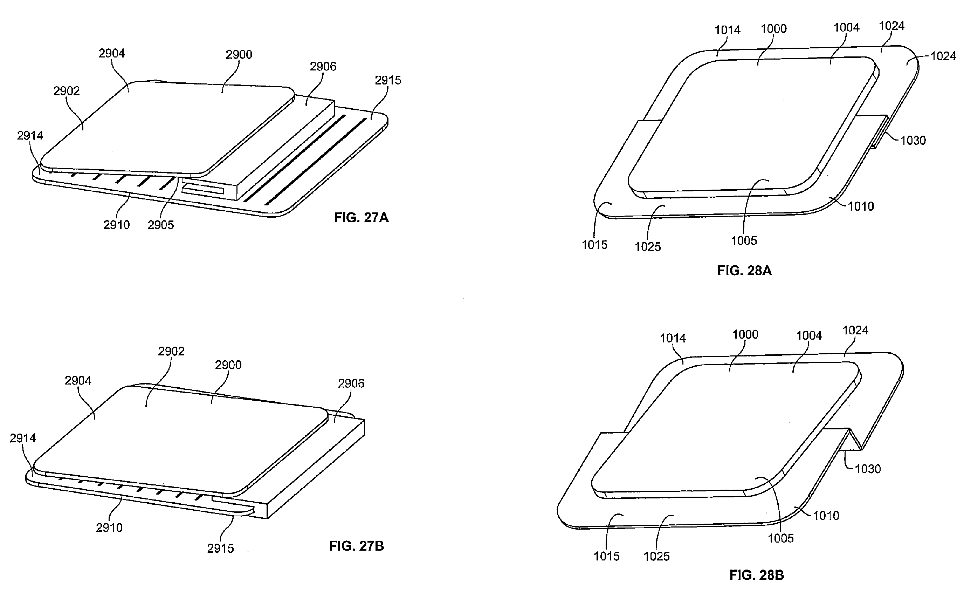

Figs. 27A and 27B illustrate a strainable skin treatment device 2900. A first side 2904 of a treatment device 2900 is anchored at a first end 2914 of a more stiff or rigid backing 2910 for example by a removable adhesive or other removable coupling element. A hook or other coupling element 2906 is coupled to the second side 2905 of the treatment device 2900. The coupling element 2906 engages or hooks onto the second side 2915 of the backing 2910. A user may manipulate the coupling element 2906 to strain the treatment device 2900 and then attach the coupling element 2906 to the second side 2915 of the backing 2910. The skin treatment device 2900 may be removably coupled to the coupling element 2906 by way of a peelable adhesive or other coupling mechanism. Thus, after straining and attaching the treatment device 2900 to the backing 2910 in a strained configuration, and after applying an adhesive side 2902 of the strained device 2900 to skin to be treated, the backing 2910 and coupling mechanism or hook 2906 may be released from the device 2900 by peeling apart from the device 2900.

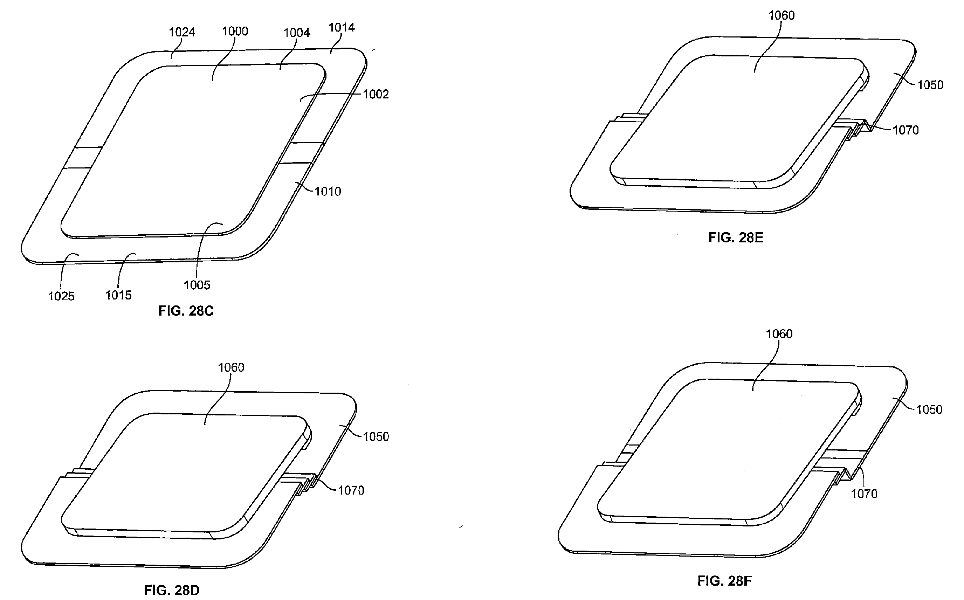

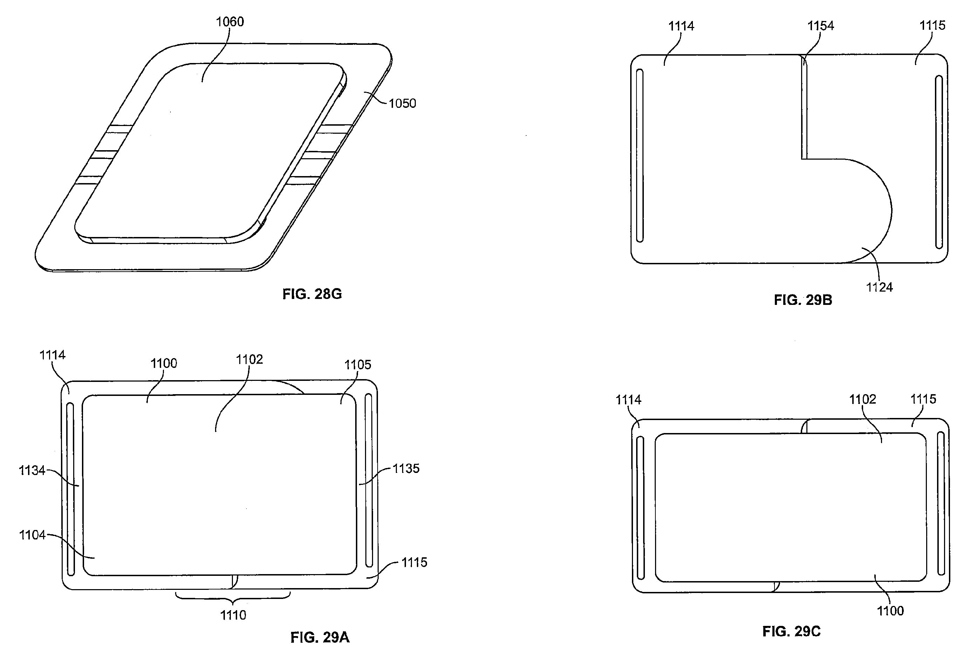

Figs. 28A to 28C illustrate a strainable skin treatment device 1000.The skin treatment device 1000 is coupled, for example with a peelable adhesive or other coupling mechanism, on opposing sides 1004, 1005 adjacent opposing sides1014, 1015 of a straining backing 1010 or applicator 1010. The backing 1010 is shown in a folded configuration in Fig. 28A, where a portion of the backing 1010 includes a tri- fold 1030. The fold 1030 decreases the length of the backing 1010 (Fig. 28A). Margins or areas 1024, 1025 of the opposing sides 1014, 1015 extend beyond the sides 1004, 1005 of the device 1000 permitting a user to grasp or manipulate the free areas to exert and tensile or straining force on the device 1000 through the backing 1010. When a straining force is exerted, the tri-fold 1030 unfolds (Fig. 28B) and straightens (28C). The backing 1010 thus limits the strain of the device 1000. After the device 1000 is strained, an adhesive side 1002 of the strained device 1000 may be applied to skin to be treated and the backing 1010 may be removed from the device 1000 leaving the device 1000 on the skin.

As shown in Figs. 28D to 28G, a plurality of folds 1070 may be provided in a backing 1050. Each fold 1070 may correspond or provide a particular strain value or amount. A user may select numbers of folds depending on desired strain. Folds1070 may be configured to release sequentially as shown in Figs. 28E to 28G, or may be secured and releasable, for straining the dressing 1060, for example by a tape that resists unfolding of a particular fold unless removed.

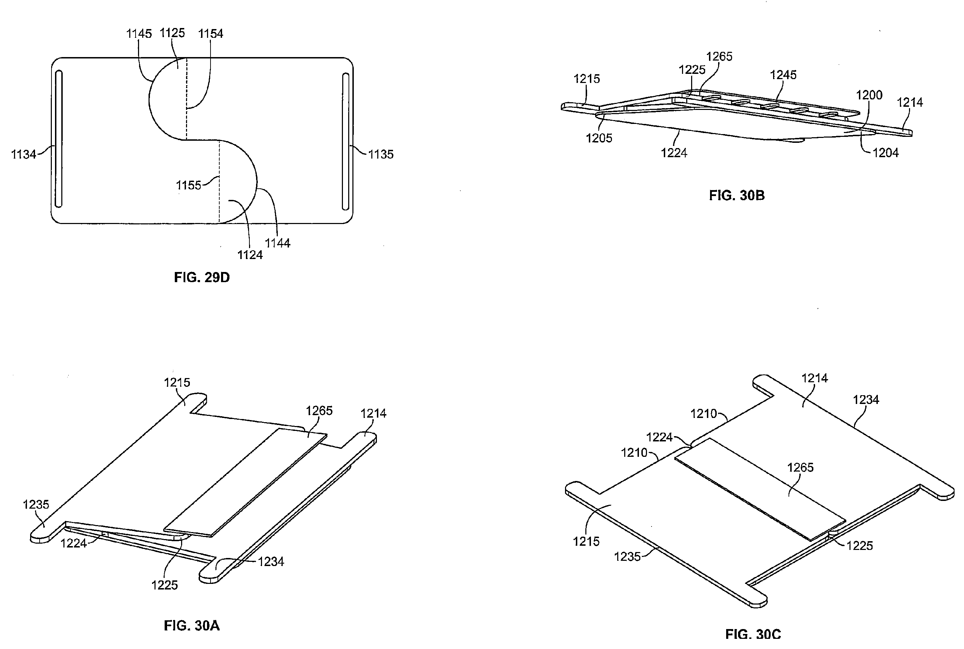

Figs. 29A to 29D illustrate a strainable skin treatment device 1100. The skin treatment device 1100 is coupled on opposing sides 1104, 1105 to sides of segments 1114, 1115 of a straining backing or applicator 1110. The device 1100 is free from to the backing 1110 in a straining zone between the opposing sides 1104, 1105. The backing 1110 comprises segments 1114, 1115 having middle tab portions 1124, 1125 that overlap in the unstrained configuration (Figs. 29A and 29B) wherein the backing 1110 has a first length. Margins or areas 1134, 1135 of sides of segments 1114, 1115 extend beyond the sides 1104, 1105 of the device 1100 permitting a user to grasp the free areas to exert a tensile or straining force on the device 1100. When a straining force is exerted, the tabs 1124, 1125 of segments 1114, 1115 separate. After separation, when released, the edges 1144, 1145 of the tabs 1124, 1125 engage by overlapping opposing edge portions1155, 1154 of segments 1115, 1114 respectively, maintaining the backing in a lengthened configuration and thus maintaining the strain in the device 1100. (Figs. 29C and 29D). After the device 1100 is strained, an adhesive side 1102 of the strained device 1100 may be applied to skin to be treated and the backing 1110 may be removed from the device 1100 leaving the device 1100 on the skin.

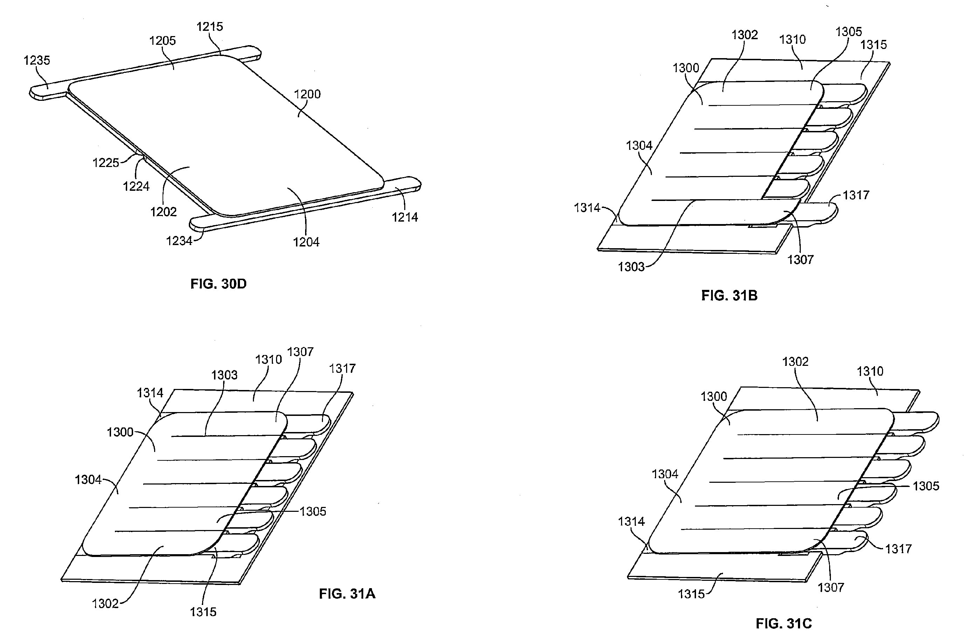

Figs. 30A to 30D illustrate a strainable skin treatment device 1200. The skin treatment device 1200 is coupled on opposing sides 1204, 1205 to sides of segments 1214, 1215 of a straining backing or applicator 1210. The device 1200 is free from to the backing 1210 in a straining zone between the opposing sides 1204, 1205. The backing 1210 comprises segments 1214, 1215 having middle edges 1224, 1225 that overlap in the unstrained configuration (Figs. 30A and 30B) wherein the backing 1210 has a first length. Handle elements 1234, 1235 of sides of segments 1214, 1215 permit a user to grasp and manipulate the segments 1214, 1215 to exert a tensile or straining force on the device 1200. When a straining force is exerted, the edges 1224, 1225 of segments 1214, 1215 separate. After separation, when released , the edges 1224, 1225 of each have features that engage with each other, maintaining the backing 1210 in a lengthened configuration and thus maintaining the strain in the device 1200. (Figs. 30C and 30D). The features may be, for example a mortise and tenon (1244) type of joint. A guide 1265 on the segment 1215 may also guide the edges 1224, 1225 in to an aligned engagement when the device 1200 is strained. After the device 1200 is strained, the adhesive side 1202 of the strained device 1200 may be applied to skin to be treated and the backing 1210 may be removed from the device 1200 leaving the device 1200 on the skin.

Figs. 31A to 31C illustrate a strainable skin treatment device 1300. A first side 1304 of a treatment device 1300 is anchored at a first side 1314 of a more stiff or rigid backing 1310 for example by a removable adhesive or other removable coupling element. The treatment device 1300 includes slits or breaks 1303 that form fingers 1307. The backing 1310 includes movable fingers 1317 that are extendable away from first end 1314 of backing 1310. The fingers 1307 of the device 1300 are each attached to a corresponding movable finger 1317 of the backing 1310 at a second side 1315 of the backing 1310. Each of the movable fingers 1317 may be extended away from side 1314 to strain a corresponding finger 1307 of the device. A user may select one or more or all fingers 1307 to be strained. Each finger 1307 may also be variably strained to a desired degree. Fig. 31A illustrates an unstrained device 1310 on the backing. Fig. 31B illustrates an individual finger 1307 strained. Fig. 31C illustrates all fingers 1307 strained. The skin treatment device 1300 may be removably coupled to the side1314 of the backing 1310 and/or edges 1305 of the finger 1307 by way of a peelable adhesive or other coupling mechanism. Thus, after straining and after applying the adhesive side 1302 of the strained device 1300 to skin to be treated, the backing 1310 including the fingers 1317 may be released from the device 1300 for example by peeling apart from the device 1300.

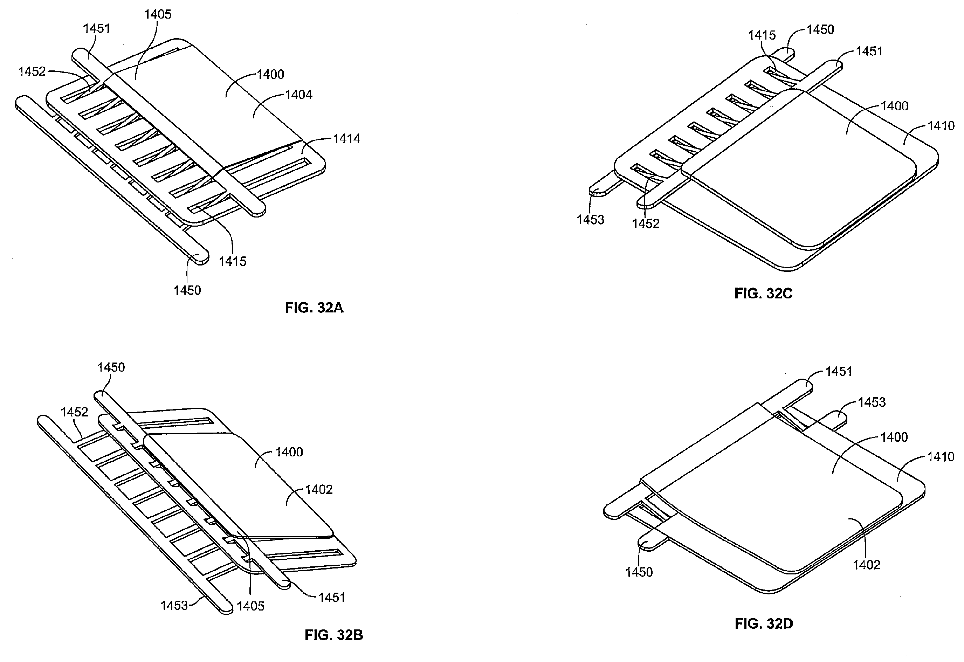

Figs. 32A and 32B illustrate a strainable skin treatment device 1400. The skin treatment device 1400 is coupled on a first side 1404 to a corresponding first side 1414 of a stiffer or more rigid backing 1410. A straining element 1450 is coupled to the second side 1405 of the treatment device 1400. The straining element 1450 comprises a first bar or handle 1451 that is coupled to the second side 1405 of the treatment device 1400, for example, by way of an adhesive or other coupling element. The straining element 1450 further comprises connecting bars 1452 that extend through slots 1415 in rigid backing 1410 and connect the first bar or handle 1451 to a second bar or handle 1453. A user may use the straining element 1450 to strain the treatment device 1400 by grasping the second handle or bar 1453 and sliding the connecting bars 1452 through the slots 1415 (Fig. 32B). The skin treatment device 1400 and the backing 1410 may be removably coupled on the first sides 1404, 1414 by way of a peelable adhesive or other coupling mechanism. Thus, after straining and after applying the adhesive side 1402 of the strained device 1400 to skin to be treated, the backing 1410 may be released from the device 1400 by peeling the backing 1410 from the device 1400.

Figs. 32C and 32D illustrate the strainable skin treatment device 1400 and rigid backing 1410 of Figs. 32A and 32B. A user may use the straining element 1450 to strain the treatment device1400 by grasping the handle or bar 1453 and rotating it so that the connecting bars 1452 rotate with in the slot 1415 as shown in Fig. 32D. After straining and after applying the adhesive side 1402 of the strained device 1400 to skin to be treated, the backing 1410 may be released from the device 1400 by peeling the backing 1410 from the device 1400.

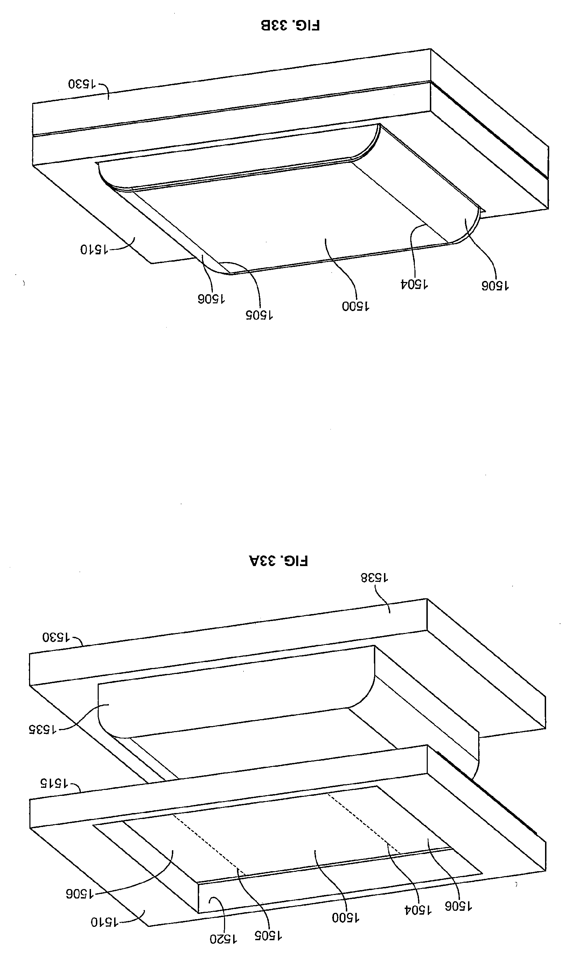

Figs. 33A to 33B illustrate a method and device for straining a skin treatment device 1500. A strainable skin treatment device 1500 is coupled over a window 1520 of a frame 1510 interfacing side 1515 of the frame 1510. The device 1500 may include extension sheets 1506 coupled at sides 1504, 1505 of the device 1500. The extension sheets 1506 may be flexible and relatively less elastic or inelastic than device 1500. A straining device 1530 comprises a protruding element 1535 on a handle1538 where the protruding element 1535 is shaped to fit within the window 1520 of the frame 1510. In use, the protruding element 1535 is placed between adjacent the treatment device 1500 and is used to push the treatment device 1500 through the window 1520, thereby straining the treatment device 1500. The handle 1538 and frame 1510 may be used together to apply the strained device 1500 to skin to be treated.

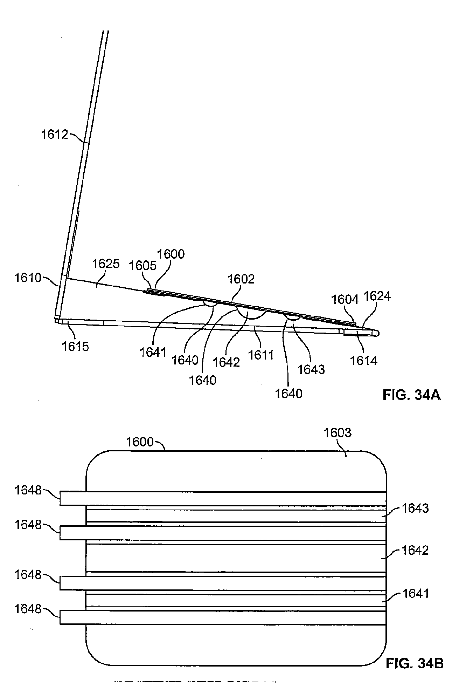

Fig. 34A illustrates a strainable dressing 1600 with an applicator or tensioning device 1610 that is described in detail in U.S 20130012858 13/345,524. The dressing 1600 may be strained to different distances to accommodate for multiple forces and tensile strengths within the dressing. Different forces may be desired at different locations, areas or parts of the dressing. The dressing 1600 is illustrated in an unstrained configuration in Fig 34A. A flexible, relatively inelastic anchor sheet 1624 couples a first side 1604 of the device 1600 of a free side 1614 of a base support 1611 of the applicator 1610. A straining sheet 1625 couples a second side 1605 of the device 1600 to the movable cover 1612 which is pivotably or hingedly coupled the side 1615 of the base support 1611. When the cover 1612 is pivoted and opened, it acts to strain the device 1600. A skin adhesive is applied to the skin adhesive side 1602 of the device 1600. A strain limiting structure 1640 is coupled to the device 1600, e.g., the opposing back surface 1603 of the device 1600. The strain limiter 1640 comprises a plurality of strain limiting elements 1641, 1642, 1643. Each strain limiting element may provide a different degree of strain limitation. According to a variation, a first central strain limiter 1642 may permit a predetermined amount of strain at a central location. Adjacent strain limiters1641, 1643 may permit a predetermined amount of strain that may or may not be different and may or may not be more or less than the amount of central strain. According to a variation, the adjacent strain limiters 1641, 1643 permit a lesser amount of strain than the central strain limiter 1642. Varied strain at different locations may be selected or provided on different dressings to provide a variety of dressings that may be targeted to particular body locations or applications of use.

According to a variation, in order to reduce irritation around the edges or to focus compressive forces near a wound or treatment site, the dressing may be strained to a greater degree, or force towards the middle of the dressing. Also forces and tensile strengths may be controlled towards the edges of the dressings within the dressing.

The strain limiters may be constructed of a flexible less elastic, as compared to the dressing material, such as an LDPE that limits strain between opposing locations where the strain limiter is coupled to the dressing. The strain limiting elements 1641, 1642, 1643 may be attached to the back surface of the dressing with a tape 1648 such as KAPTON® tape, or peelable adhesive. An acrylic adhesive may be used to attach the LDPE to the back side of the kapton. After the dressing is strained and applied to a subject, the strain limiter may or may not be released.

The applications of the disclosed invention discussed above are not limited to certain treatments or regions of the body, but may include any number of other treatments and areas of the body. Modification of the above-described devices for carrying out the invention, and variations of aspects of the invention that are obvious to those of skill in the arts are intended to be within the scope of this disclosure. Moreover, various combinations of aspects between examples are also contemplated and are considered to be within the scope of this disclosure as well.

## Claims

1. A skin treatment device comprising:
a first layer (100) comprising an elastic material prestretched to a predetermined strain level and;
a second layer coupled to the first layer (100) and comprising a brace (110) configured to maintain the first layer (100) at the predetermined strain level while the device is attached to a skin layer (10) of a subject, the brace (110) comprising a plurality of second layer openings (112) positionable for treatment of skin therethrough; and
a securing element configured to secure the device to skin of a subject during treatment through said plurality of second layer openings (112), wherein the security element comprises an adhesive (104).

2. The skin treatment device of claim 1, wherein the first layer (100) comprises a plurality of elastic material openings (108) through the elastic material configured to be aligned with the plurality of second layer openings (112) for treatment through the aligned openings (108, 112).

3. The skin treatment device of claim 1, wherein the brace (110) further comprises a mating element (116) configured to secure the first layer (100) in a strained configuration on the brace (110).

4. The skin treatment device of claim 1, wherein the brace (110) is rigid.

5. The skin treatment device of claim 1, wherein the brace (110) comprises a plurality of segments bendable with respect to another of the plurality of segments to provide a variable contour of the brace (110).

6. The skin treatment device of claim 5, wherein the brace (110) is configured to be bendable in a plurality of directions.

7. The skin treatment device of claim 5, wherein each of the plurality of segments are bendable in a plurality of directions.

8. The skin treatment device of any one of claims 1 to 3, wherein the brace (110) is malleable.

9. The skin treatment device of claim 1, wherein the second layer comprises a mask (128) having a pattern of openings (129).

10. The skin treatment device of claim 9, wherein the first layer (100) comprises a pattern of treatment openings (108).

11. The skin treatment device of claim 1, further comprising a connection element configured to connect the device to an energy emitting skin treatment device.

12. The skin treatment device of claim 9, wherein the plurality of second layer openings (112) comprises a pattern of openings that corresponds to the pattern of openings (129) of the mask (128).

13. The skin treatment device of claim 1, wherein the second layer is removable from the first layer (100).

14. The skin treatment device of claim 2, wherein the elastic material openings (108) are reinforced by reinforcement structures (124, 125) comprising open or closed perimeter structures surrounding the elastic material openings (108).

15. The skin treatment device of any one of claims 1 to 14, wherein the brace (110) comprises a flexible but incompressible material configured to resist a compression load per millimeter width of at least about 0.1 Newton, or 0.1 Newton, or 0.2 Newton, or 0.3 Newton, or 0.4 Newton, or 0.5 Newton.

## Patentansprüche

1. Hautbehandlungsvorrichtung, umfassend:
eine erste Schicht (100), umfassend ein auf ein vorbestimmtes Spannungsniveau vorgestrecktes, elastisches Material und;
eine zweite Schicht, welche an die erste Schicht (100) gekoppelt ist und eine Versteifung (110) umfasst, welche dazu eingerichtet ist, die erste Schicht (100) auf dem vorgegebenen Spannungsniveau zu halten, während die Vorrichtung auf einer Hautschicht (10) einer Behandlungsperson befestigt wird, wobei die Versteifung (110) eine Vielzahl von Zweitschichtöffnungen (112) umfasst, welche zur dadurch erfolgenden Hautbehandlung positionierbar ist; und
ein Befestigungselement, welches dazu eingerichtet ist, die Vorrichtung während einer durch die Vielzahl von Zweitschichtöffnungen (112) erfolgenden Behandlung auf einer Haut einer Behandlungsperson zu befestigen, wobei das Befestigungselement einen Klebstoff (104) umfasst.

2. Hautbehandlungsvorrichtung gemäß Anspruch 1, wobei die erste Schicht (100) eine Vielzahl von elastischen Materialöffnungen (108) durch das elastische Material umfasst, welche dazu eingerichtet sind, sich mit der Vielzahl von Zweitschichtöffnungen (112) zur Behandlung durch die sich deckenden Öffnungen (108, 112) zu decken.

3. Hautbehandlungsvorrichtung gemäß Anspruch 1, wobei die Versteifung (110) ferner ein Steckelement (116) umfasst, welches dazu eingerichtet ist, die erste Schicht (100) in einer angespannten Anordnung auf der Versteifung (110) zu befestigen.

4. Hautbehandlungsvorrichtung gemäß Anspruch 1, wobei die Versteifung (110) starr ist.

5. Hautbehandlungsvorrichtung gemäß Anspruch 1, wobei die Versteifung (110) eine Vielzahl von Segmenten aufweist, welche bezüglich eines anderen der Vielzahl von Segmenten biegsam ist, um eine veränderliche Kontur der Versteifung (110) bereitzustellen.

6. Hautbehandlungsvorrichtung gemäß Anspruch 5, wobei die Versteifung (110) dazu eingerichtet ist, in einer Vielzahl von Richtungen biegsam zu sein.

7. Hautbehandlungsvorrichtung gemäß Anspruch 5, wobei ein jedes der Vielzahl von Segmenten in einer Vielzahl von Richtungen biegsam ist.

8. Hautbehandlungsvorrichtung gemäß einem jeden der Ansprüche 1 bis 3, wobei die Versteifung (110) verformbar ist.

9. Hautbehandlungsvorrichtung gemäß Anspruch 1, wobei die zweite Schicht eine Maske (128) umfasst, welche ein Raster von Öffnungen (129) besitzt.

10. Hautbehandlungsvorrichtung gemäß Anspruch 9, wobei die erste Schicht (110) ein Raster von Behandlungsöffnungen (108) umfasst.

11. Hautbehandlungsvorrichtung gemäß Anspruch 1, ferner umfassend ein Verbindungselement, welches dazu eingerichtet ist, die Vorrichtung mit einer energieabgebenden Hautbehandlungsvorrichtung zu verbinden.

12. Hautbehandlungsvorrichtung gemäß Anspruch 9, wobei die Vielzahl von Zweitschichtöffnungen (112) ein Raster von Öffnungen umfasst, welches sich mit dem Raster der Öffnungen (129) der Maske (128) deckt.

13. Hautbehandlungsvorrichtung gemäß Anspruch 1, wobei die zweite Schicht von der ersten Schicht (100) entfernbar ist.

14. Hautbehandlungsvorrichtung gemäß Anspruch 2, wobei die elastischen Materialöffnungen (108) durch Verstärkungsstrukturen (124, 125) verstärkt sind, welche offene oder geschlossene Umfangsstrukturen umfassen, welche die elastischen Materialöffnungen (108) umgeben.

15. Hautbehandlungsvorrichtung gemäß einem jeden der Ansprüche 1 bis 14, wobei die Versteifung (110) ein flexibles, aber nicht komprimierbares Material umfasst, welches dazu eingerichtet ist, einer Kompressionslast je Millimeter Breite von zumindest etwa 0.1 Newton, oder 0.1 Newton, oder 0.2 Newton, oder 0.3 Newton, oder 0.4 Newton, oder 0.5 Newton zu widerstehen.

## Revendications

1. Dispositif de traitement de la peau comprenant :
une première couche (100) comprenant une matière élastique préétirée à un niveau de contrainte prédéterminé ; et
une deuxième couche couplée à la première couche (100) et comprenant une cale (110) configurée pour maintenir la première couche (100) au niveau de contrainte prédéterminé pendant que le dispositif est attaché à une couche de peau (10) d'un sujet, la cale (110) comprenant une pluralité d'ouvertures de deuxième couche (112) pouvant être positionnées pour permettre le traitement de la peau à travers elles ; et
un élément de fixation configuré pour assujettir le dispositif sur la peau d'un sujet pendant le traitement à travers ladite pluralité d'ouvertures de deuxième couche (112), dans lequel l'élément de sécurité comprend un adhésif (104).

2. Dispositif de traitement de la peau selon la revendication 1, dans lequel la première couche (100) comprend une pluralité d'ouvertures de matière élastique (108) dans la matière élastique, configurées pour être alignées avec la pluralité d'ouvertures de deuxième couche (112) pour permettre le traitement à travers les ouvertures alignées (108, 112).

3. Dispositif de traitement de la peau selon la revendication 1, dans lequel la cale (110) comprend en outre un élément homologue (116) configuré pour assujettir la première couche (100) dans une configuration contrainte sur la cale (110).

4. Dispositif de traitement de la peau selon la revendication 1, dans lequel la cale (110) est rigide.

5. Dispositif de traitement de la peau selon la revendication 1, dans lequel la cale (110) comprend une pluralité de segments pouvant être pliés par rapport à un autre segment de la pluralité de segments pour donner un contour variable à la cale (110).

6. Dispositif de traitement de la peau selon la revendication 5, dans lequel la cale (110) est configurée pour pouvoir être pliée dans une pluralité de directions.

7. Dispositif de traitement de la peau selon la revendication 5, dans lequel chacun des segments peut être plié dans une pluralité de directions.

8. Dispositif de traitement de la peau selon l'une quelconque des revendications 1 à 3, dans lequel la cale (110) est malléable.

9. Dispositif de traitement de la peau selon la revendication 1, dans lequel la deuxième couche comprend un masque (128) ayant un motif formé par des ouvertures (129).

10. Dispositif de traitement de la peau selon la revendication 9, dans lequel la première couche (100) comprend un motif formé par des ouvertures de traitement (108).

11. Dispositif de traitement de la peau selon la revendication 1, comprenant en outre un élément de connexion configuré pour connecter le dispositif à un dispositif de traitement de la peau émettant de l'énergie.

12. Dispositif de traitement de la peau selon la revendication 9, dans lequel la pluralité d'ouvertures de deuxième couche (112) comprend un motif d'ouvertures qui correspond au motif d'ouvertures (129) du masque (128).

13. Dispositif de traitement de la peau selon la revendication 1, dans lequel la deuxième couche peut être retirée de la première couche (100).

14. Dispositif de traitement de la peau selon la revendication 2, dans lequel les ouvertures de matière élastique (108) sont renforcées par des structures de renforcement (124, 125) comprenant des structures de périmètre ouvert ou fermé qui entourent les ouvertures de matière élastique (108).

15. Dispositif de traitement de la peau selon l'une quelconque des revendications 1 à 14, dans lequel la cale (110) comprend un matériau flexible mais incompressible configuré pour résister à une charge de compression par largeur d'un millimètre au moins égale à environ 0,1 newton, ou 0,1 newton, ou 0,2 newton, ou 0,3 newton, ou 0,4 newton, ou 0,5 newton.
